(19)

**Europäisches Patentamt**
**European Patent Office**
**Office européen des brevets**

(11) **EP 4 470 693 A1**

(12) **EUROPEAN PATENT APPLICATION**
published in accordance with Art. 153(4) EPC

(43) Date of publication:
**04.12.2024 Bulletin 2024/49**

(21) Application number: **23766490.9**

(22) Date of filing: **17.02.2023**

(51) International Patent Classification (IPC):
**B22D 11/16** (2006.01)   **G01B 17/00** (2006.01)

(52) Cooperative Patent Classification (CPC):
**B22D 11/16; G01B 17/00**

(86) International application number:
**PCT/JP2023/005639**

(87) International publication number:
**WO 2023/171311 (14.09.2023 Gazette 2023/37)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **10.03.2022 JP 2022036855**

(71) Applicant: **JFE Steel Corporation
Tokyo 100-0011 (JP)**

(72) Inventors:
• **NISHIZAWA, Yuji**
  **Tokyo 100-0011 (JP)**
• **MIKURIYA, Tomoaki**
  **Tokyo 100-0011 (JP)**
• **ISOZAKI, Kenji**
  **Tokyo 100-0011 (JP)**
• **TAKENAKA, Shudo**
  **Tokyo 100-0011 (JP)**
• **WATANABE, Hitoshi**
  **Tokyo 100-0011 (JP)**

(74) Representative: **Haseltine Lake Kempner LLP
Bürkleinstrasse 10
80538 München (DE)**

(54) **SOLIDIFICATION POSITION MEASUREMENT DEVICE, SOLIDIFICATION POSITION MEASUREMENT METHOD, METAL MATERIAL QUALITY MANAGEMENT METHOD, CASTING EQUIPMENT, METAL MATERIAL MANUFACTURING EQUIPMENT, AND METAL MATERIAL MANUFACTURING METHOD**

(57)     An object of the present invention is to make it possible to continuously and automatically measure a solidification position of a cast-piece by preventing damage and burning of an electromagnetic ultrasonic sensor due to a decrease in lift-off caused by a fluctuation in the thickness of the cast-piece, and preventing a decrease in sensitivity of the electromagnetic ultrasonic sensor due to an increase in lift-off. A solidification position measuring apparatus includes: electromagnetic ultrasonic sensors 7 and 8 installed outside a cast-piece 1; a sensor lifting and lowering unit configured to adjust a gap between the cast-piece 1 and the electromagnetic ultrasonic sensor 7; a gap measuring unit configured to measure the gap between the cast-piece 1 and the electromagnetic ultrasonic sensor 7; a measuring unit configured to estimate a solidification position in the cast-piece 1 from an output of the electromagnetic ultrasonic sensor 7; and a drive control unit configured to drive and control the sensor lifting and lowering unit and the gap measuring unit. The drive control unit is configured to control a position of the electromagnetic ultrasonic sensor 7 by the sensor lifting and lowering unit based on the gap measured by the gap measuring unit.

FIG.2

## Description

Field

The present invention relates to a solidification position measuring apparatus, a solidification position measuring method, a metal material quality management method, a casting facility, a metal material manufacturing facility, and a metal material manufacturing method. Background

A solidification position, particularly the final solidification position, also called crater end, of a cast-piece, particularly a cast-piece of steel is an important indicator for quality and productivity. For example, when a casting speed is increased to increase productivity, the final solidification position moves to the downstream side in a casting direction. When the final solidification position exceeds a range of a cast-piece support roll, the cast-piece swells due to the action of a static iron pressure, and problems such as deterioration of internal quality and casting stop due to huge bulging occur. In addition, in a soft reduction operation for a center segregation reduction of the cast-piece, it is necessary to control the casting speed and the amount of secondary cooling water and appropriately perform the reduction so that the final solidification position is located in a soft reduction zone. Note that it is known that the final solidification position in the cast-piece is not uniform in a width direction and fluctuates with time. It is known that the difference in the final solidification position in the width direction and the shape of the final solidification position are also important factors in terms of the quality of the cast-piece.

In order to meet these requirements, various methods have been proposed in order to grasp a solidification state of the cast-piece and grasp the final solidification position. For example, there is a method in which metal nails are driven to the cast-piece, and it is confirmed whether there are a molten layer and a fluid layer in the central portion. In addition, there is a method of performing heat transfer calculation in a solidification process and estimating the final solidification position. In addition, there is a method of continuously measuring a surface temperature, correcting heat transfer calculation, and improving accuracy. Further, as a method of directly measuring the solidification position online, there is a method of directly measuring the solidification position using an electromagnetic ultrasonic sensor. For example, Patent Literature 1 discloses a method in which a final solidification position is detected by a transverse ultrasonic wave, heat transfer calculation is corrected, and a solidification completion position is obtained from a propagation time of a longitudinal ultrasonic wave.

Citation List

Patent Literature

Patent Literature 1: JP 2010-5700 A
Patent Literature 2: JP H10-122844 A

Summary

Technical Problem

As described above, there is a strong need to grasp the solidification position and the shape, and measurement using electromagnetic ultrasonic waves has been performed, but the measurement has not been performed automatically. There are several problems that have been obstacles to automatic measurement, and the problems include a lift-off fluctuation caused by a slab thickness fluctuation or the like and the difficulty of automatic determination due to a decrease/fluctuation in sensitivity.

Specifically, in the solidification position measuring method using the electromagnetic ultrasonic sensor, a solidification position shape can be calculated from the detection/calibration of solidification and non-solidification and a longitudinal wave propagation time using the transverse ultrasonic wave and the longitudinal ultrasonic wave. This electromagnetic acoustic transducer is a method capable of transmitting and receiving ultrasonic waves in a non-contact manner, but a gap between a measurement target and the electromagnetic ultrasonic sensor needs to be about 5 mm, and this gap (lift-off) fluctuates in actual operation. For example, the lift-off fluctuates due to a fluctuation in the thickness of the cast-piece, a local or periodic fluctuation in the thickness due to bulging, or the like.

For example, when solidification occurs in an upper part of a continuous casting facility due to rapid deceleration, casting stop, or the like, a thick cast-piece may come. In this case, there is a possibility that the electromagnetic ultrasonic sensor comes into contact with the cast-piece and is damaged or burned. In addition, if the electromagnetic ultrasonic sensor is damaged or dropped due to this contact, the electromagnetic ultrasonic sensor may be caught in a roll to cause a flaw of the cast-piece, damage the casting roll, and further cause a flaw continuously of the cast-piece due to a roll flaw, leading to serious trouble.

On the other hand, for example, in a situation where the static iron pressure disappears at the end of casting, the thickness of the cast-piece becomes thin, the lift-off becomes large, and the sensitivity of the electromagnetic ultrasonic sensor cannot be secured.

That is, when the thickness of the cast-piece increases and the lift-off decreases, contact between the electromagnetic ultrasonic sensor and the cast-piece, and the like may occur, and the electromagnetic ultrasonic sensor may be damaged. Conversely, when the thickness of the cast-piece decreases and the lift-off becomes too large, the sensitivity of the electromagnetic ultrasonic sensor cannot be secured, so that it has not been possible to perform automatic and continuous solidification position measurement.

In addition, problems related to sensitivity and waveform distortion of electromagnetic ultrasonic waves have also been major factors that hinder automation. Since the transmission/reception sensitivity of the electromagnetic ultrasonic waves is low, an S/N of transmitted/received ultrasonic waves cannot be kept, and there has been a problem that manual analysis is required, or the transmission/reception waveform is distorted due to the influence of the surface temperature or the like, and automatic measurement becomes difficult.

The present invention has been made in view of the above problems, and an object of the present invention is to provide a solidification position measuring apparatus, a solidification position measuring method, a metal material quality management method, a casting facility, a metal material manufacturing facility, and a metal material manufacturing method that make it possible to continuously and automatically measure a solidification position of a cast-piece by preventing damage and burning of an electromagnetic ultrasonic sensor due to a decrease in lift-off caused by a fluctuation in the thickness of the cast-piece, preventing a decrease in sensitivity of the electromagnetic ultrasonic sensor due to an increase in lift-off, and further automatically measuring a reception signal by greatly improving an S/N of a transmitting/receiving waveform.

Solution to Problem

(1) To solve the above-described problem and achieve the object, a solidification position measuring apparatus according to the present invention measures a solidification position of a cast-piece, and includes: an electromagnetic ultrasonic sensor installed outside the cast-piece; a sensor lifting and lowering unit configured to adjust a gap between the cast-piece and the electromagnetic ultrasonic sensor; a gap measuring unit configured to measure the gap between the cast-piece and the electromagnetic ultrasonic sensor; a computing unit configured to estimate the solidification position in the cast-piece from an output of the electromagnetic ultrasonic sensor; and a drive control unit configured to drive and control the sensor lifting and lowering unit and the gap measuring unit, wherein the drive control unit is configured to control a position of the electromagnetic ultrasonic sensor by the sensor lifting and lowering unit based on the gap measured by the gap measuring unit.

(2) Moreover, the solidification position measuring apparatus according to (1) of the present invention includes a sensor traveling unit configured to move, outside the cast-piece, the electromagnetic ultrasonic sensor in a plane parallel to a horizontal plane of the cast-piece.

(3) Moreover, in the above-described solidification position measuring apparatus according to (1) or (2) of the present invention, the gap measuring unit is configured to shoot water to a measurement target, propagate an ultrasonic wave in the shot water, and measure a distance from ultrasonic wave generating unit to the measurement target based on a propagation time of a reflection signal of the ultrasonic wave.

(4) Moreover, in the above-described solidification position measuring apparatus according to any one of (1) to (3) of the present invention, a transmission/reception signal of the electromagnetic ultrasonic sensor is a chirp signal whose frequency changes with time, and the computing unit is configured to perform computing processing including a phase operation on the chirp signal.

(5) Moreover, in the above-described solidification position measuring apparatus according to (4) of the present invention, the computing unit is configured to detect, after the computing processing, a waveform of a transmitted wave and/or a reflected wave at a time gate including the transmitted wave and/or the reflected wave, and compute a propagation time using a time difference of the transmitted wave and/or the reflected wave whose waveform is detected.

(6) Moreover, in the above-described solidification position measuring apparatus according to any one of (1) to (5) of the present invention, the electromagnetic ultrasonic sensor is configured to use a Halbach array magnet.

(7) To solve the above-described problem and achieve the object, a solidification position measuring method according to the present invention measures a solidification position of a cast-piece, and includes: a gap measuring step of measuring a gap between the cast-piece and an electromagnetic ultrasonic sensor; a gap determining step of determining whether or not the gap measured is within a prescribed range; a gap adjusting step of adjusting the gap based on the gap measured when the gap is not within the prescribed range; and a computing step of estimating the solidification position in the cast-piece using the electromagnetic ultrasonic sensor when the gap is within the prescribed range.

(8) Moreover, the above-described solidification position measuring method according to (7) of the present invention includes a moving step of moving the electromagnetic ultrasonic sensor in a plane parallel to a horizontal plane of the cast-piece.

(9) Moreover, in the above-described solidification position measuring method according to (7) or (8) of the present invention, the electromagnetic ultrasonic sensor is configured to use a Halbach array magnet, a transmitting /receiving signal of the electromagnetic ultrasonic sensor is a chirp signal whose frequency changes with time, and the computing step performs computing processing including a phase operation on the chirp signal.

(10) To solve the above-described problem and achieve the object, a metal material quality management method according to the present invention includes controlling a manufacturing condition of a next process from a solidification position of a cast-piece measured using the solidification position measuring method according to any one of (7) to (9).

(11) To solve the above-described problem and achieve the object, a metal material manufacturing facility according to the present invention includes the solidification position measuring apparatus according to any one of (1) to (6), wherein a metal material is manufactured while the solidification position of the cast-piece is measured by the solidification position measuring apparatus.

(12) To solve the above-described problem and achieve the object, a casting facility according to the present invention includes the solidification position measuring apparatus according to any one of (1) to (6), wherein a cast-piece is manufactured while the solidification position of the cast-piece is measured by the solidification position measuring apparatus.

(13) To solve the above-described problem and achieve the object, a metal material manufacturing facility according to the present invention includes the casting facility according to (12), wherein a metal material is manufactured from a cast-piece manufactured by the casting facility.

(14) To solve the above-described problem and achieve the object, a metal material manufacturing method according to the present invention includes manufacturing a metal material while measuring a solidification position of a cast-piece using the solidification position measuring method according to any one of (7) to (9).

Advantageous Effects of Invention

According to the solidification position measuring apparatus, the solidification position measuring method, the metal material quality management method, the casting facility, the metal material manufacturing facility, and the metal material manufacturing method according to the present invention, it is possible to continuously and automatically measure the solidification position of the cast-piece by preventing the damage and burning of the electromagnetic ultrasonic sensor due to a decrease in lift-off caused by a fluctuation in the thickness of the cast-piece, preventing a decrease in sensitivity of the electromagnetic ultrasonic sensor due to an increase in lift-off, and further automatically measuring the reception signal by greatly improving the S/N of the transmission/reception waveform.

Brief Description of Drawings

FIG. 1 is a diagram illustrating a schematic configuration of a continuous casting facility including a solidification position measuring apparatus according to an embodiment of the present invention.
FIG. 2 is a schematic diagram illustrating a configuration of the solidification position measuring apparatus according to the embodiment.
FIG. 3 is a flowchart illustrating a procedure of measurement control processing of a solidification position by the solidification position measuring apparatus.
FIG. 4 is a flowchart illustrating a procedure of the measurement control processing of a solidification position in a specific position measurement mode by the solidification position measuring apparatus.
FIG. 5 is a diagram illustrating an example of an arrangement modification of a boom, an electromagnetic ultrasonic sensor, and a water column distance meter.
FIG. 6 is a diagram illustrating an example of an arrangement modification of the boom, the electromagnetic ultrasonic sensor, and the water column distance meter.
FIG. 7 is a cross-sectional view illustrating a configuration of the water column distance meter.
FIG. 8 is an explanatory diagram for explaining generation of a longitudinal wave by the electromagnetic ultrasonic sensor.

FIG. 9 is an explanatory diagram for explaining generation of a transverse wave by the electromagnetic ultrasonic sensor.

FIG. 10 is a diagram illustrating an example of arrangement of magnets, a coil for longitudinal wave, and a coil for transverse wave in the electromagnetic ultrasonic sensor.

FIG. 11 is a diagram illustrating a modification in which auxiliary magnets are added to the magnets of the electromagnetic ultrasonic sensor illustrated in FIG. 10.

FIG. 12 is a diagram illustrating reception signal waveforms depending on the presence or absence of computing processing (chirp pulse compression processing) with the magnets of the electromagnetic ultrasonic sensor arranged in a Halbach array.

FIG. 13 is a diagram illustrating a reception waveform obtained by using the Halbach array magnets illustrated in FIG. 11 and executing the chirp pulse compression and a reception waveform in a case where a chirp pulse compression signal and the normal magnets illustrated in FIG. 10 are used.

FIG. 14 is an explanatory diagram for explaining transverse wave measurement.

FIG. 15 is an explanatory diagram for explaining longitudinal wave measurement.

FIG. 16 is a diagram illustrating an example of automatic measurement in the full width of a cast-piece.

FIG. 17 is a diagram illustrating an example of pass/fail determination for the cast-piece.

Description of Embodiments

A solidification position measuring apparatus, a solidification position measuring method, a metal material quality management method, a casting facility, a metal material manufacturing facility, and a metal material manufacturing method according to an embodiment of the present invention will be described with reference to the drawings. Note that the present invention is not limited to the following embodiment, and constituent elements in the following embodiment include those that can be easily replaced by those skilled in the art or those that are substantially the same. In addition, a cast-piece refers to a material obtained after a metal material is cast.

<Outline of Continuous Casting Facility>

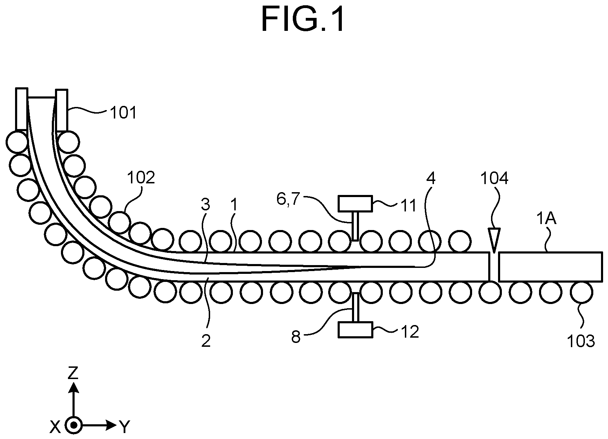

FIG. 1 is a diagram illustrating a schematic configuration of a continuous casting facility including a solidification position measuring apparatus according to the embodiment of the present invention. In addition, for ease of explanation, as an example, the "metal material" is a "steel material", the "casting facility" is a "continuous casting facility", and the "cast-piece" is a cast-piece of steel cast by the continuous casting facility. As illustrated in FIG. 1, in the continuous casting facility, molten steel injected into a mold 101 is cooled by the mold 101 to form a solidified portion 2 at a portion in contact with the mold 101, and a cast-piece 1 whose periphery is the solidified portion 2 and whose inside is an unsolidified liquid phase portion 3 is generated. Note that directions are defined in FIG. 1. X direction: cast-piece width direction, Y direction: cast-piece pulling-out direction, and Z direction: cast-piece thickness direction and lift-off direction. The same applies hereinafter.

The cast-piece 1 is pulled out below the mold 101 while being supported by a plurality of pairs of cast-piece support rolls 102 disposed to face each other below the mold 101. A secondary cooling zone including an air mist spray nozzle and a water spray nozzle (not illustrated) for spraying cooling water toward a surface of the cast-piece 1 is installed in a gap between the cast-piece support rolls 102 adjacent in a casting direction, and the cast-piece 1 is cooled in the secondary cooling zone while being pulled out to the downstream side in the casting direction, and is completely solidified up to a central portion. A position at which the cast-piece is completely solidified up to the center portion is a final solidification position called crater end. Hereinafter, the final solidification position may be referred to as a crater end position 4 or simply as a solidification position 4. The cast-piece 1 that has been solidified is cut to a predetermined length by a cast-piece cutting machine 104 installed on the downstream side of the cast-piece support roll 102, and is carried out as a cast-piece 1A by a conveying roll 103.

Here, the solidification position measuring apparatus is disposed in the continuous casting facility. The solidification position measuring apparatus includes electromagnetic ultrasonic sensors 7 and 8 disposed opposite to each other with the cast-piece 1 interposed therebetween, and a water column distance meter 6 as a gap measuring unit for measuring a gap (lift-off) between the cast-piece and the electromagnetic ultrasonic sensor 7. The electromagnetic ultrasonic sensor 8 is a sensor that transmits electromagnetic ultrasonic waves, and the electromagnetic ultrasonic sensor 7 is a sensor that receives electromagnetic ultrasonic waves. The electromagnetic ultrasonic sensors 7 and 8 transmit a transverse electromagnetic ultrasonic wave and a longitudinal electromagnetic ultrasonic wave to the cast-piece 1 and receive them via the cast-piece 1. Since the transverse electromagnetic ultrasonic wave does not pass through the liquid phase portion 3, it is possible to measure whether the cast-piece 1 is solidified or unsolidified. That is, it is possible to determine whether a measurement position is on the upstream side or the downstream side of the solidification position 4. In addition, the longitudinal electromagnetic ultrasonic wave is used to compute and estimate the solidification position 4 based on a propagation time for propagating through the cast-piece 1. The electromagnetic ultrasonic sensor 7 and the water column distance meter 6 are attached below (-Z direction) a boom 11 disposed on the +Z direction side of the cast-piece 1, and the electromagnetic ultrasonic sensor 8 is attached above (+Z direction) a boom 12 disposed on the -Z direction side of the cast-piece 1. The electromagnetic ultrasonic sensor 7 and the water column distance meter 6, and the electromagnetic ultrasonic sensor 8 are movable together with the booms 11 and 12, respectively. Each of the booms 11 and 12 can be lifted and lowered in the ±Z direction and moved in the ±X direction. A lift-off value, which is the gap between the electromagnetic ultrasonic sensor 7 and the cast-piece 1, is adjusted based on a lift-off value between the water column distance meter 6 and the cast-piece 1 measured by the water column distance meter 6. Note that the continuous casting facility here has a mechanism in which a casting thickness is controlled by controlling the position of the roll on the upper side basically with the lower side as a reference surface, the lift-off value of the electromagnetic ultrasonic sensor 8 on the lower side is fixed, and the lift-off value of the electromagnetic ultrasonic sensor 7 on the upper side is adjusted and controlled.

<Configuration of Solidification position measuring apparatus>

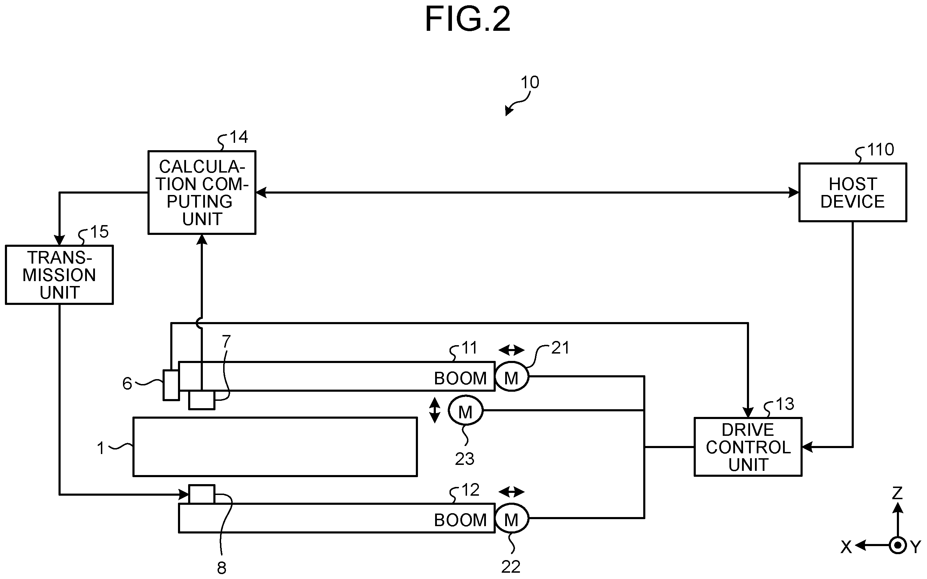

FIG. 2 is a schematic diagram illustrating a configuration of a solidification position measuring apparatus 10 according to the embodiment. As illustrated in FIG. 2, the solidification position measuring apparatus 10 is disposed such that the boom 11 to which the water column distance meter 6, which is the gap measuring unit, and the electromagnetic ultrasonic sensor 7 are attached and the boom 12 to which the electromagnetic ultrasonic sensor 8 is attached sandwich the cast-piece 1 in the Z direction (height direction). The boom 11 is lifted and lowered in the height direction by a motor 23, which is a sensor lifting and lowering unit, and is caused to travel (travel transversely) in the X direction (width direction) by a motor 21, which is a sensor traveling unit. In addition, the boom 12 is caused to travel transversely by a motor 22, which is the sensor traveling unit. Note that the sensor traveling unit moves the electromagnetic ultrasonic sensors 7 and 8 to a predetermined measurement position in a plane parallel to a horizontal plane of the cast-piece.

A drive control unit 13 acquires the lift-off value measured by the water column distance meter 6, drives and controls the motors 21 to 23 based on the lift-off value and operating information such as the cast-piece thickness and the cast-piece width sent from a host device 110, and performs travel control and lifting and lowering control of the electromagnetic ultrasonic sensors 7 and 8 via the booms 11 and 12.

A measurement computing unit 14, which is a computing unit, applies a transmission signal to the electromagnetic ultrasonic sensor 8 via a transmission unit 15 to cause the electromagnetic ultrasonic sensor 8 to transmit electromagnetic ultrasonic waves toward the cast-piece 1, induces ultrasonic waves in the cast-piece 1, applies appropriate signal processing based on the ultrasonic reception signal received by the electromagnetic ultrasonic sensor 7, estimates and computes the solidification position 4, a solidification shape, and the like, and transmits the computation result to the host device 110.

That is, in the solidification position measuring apparatus 10, the water column distance meter 6 constantly measures the lift-off value for the cast-piece 1, and based on the measurement result, the lifting and lowering control and the traverse travel control of the electromagnetic ultrasonic sensors 7 and 8 are automatically performed to automate the solidification position measurement. Note that the transverse travel enables automatic travel by the motors 21 and 22 and enables stopping at an arbitrary or previously specified position to perform measurement. For motor control, a general pulse motor, servomotor, or the like may be used, and the positional accuracy thereof is preferably about 1 mm or less.

For the height adjustment (lift-off adjustment) of the electromagnetic ultrasonic sensor 7, it is conceivable to adjust the height by controlling the lifting and lowering of only the electromagnetic ultrasonic sensor 7. However, since a driving unit for the height adjustment enters the continuous casting facility, it is preferable to install the driving unit outside the continuous casting facility to enable the lifting and lowering of the entire boom because the driving unit is under dimensional restriction (a roll gap is normally about 30 to 70 mm) and a bad environment such as steam and heat. The accuracy of lifting and lowering in the height direction is preferably about 0.1 mm. For the control, a general pulse motor, servomotor, or the like may be used. For a lifting and lowering speed and a positioning speed, a speed of about 0.1 mm/s to 1 mm/s can be exemplified. When the height adjustment is performed due to a change in the cast-piece thickness or the like as described above, the height adjustment may be designed by a speed necessary for adjusting sagging of the booms 11 and 12 due to transverse movement (tip ends are lowered by elastic deformation due to the boom's own weight) or for height adjustment due to other factors. When the speed is too slow, the lifting and lowering may not be in time and contact may occur. On the other hand, when the speed is higher than necessary, position control performance deteriorates or a motor portion becomes too large and the cost increases. Changes in casting process typically proceed in the time scale of a few minutes or in a few minutes of delay. Therefore, it is preferable to perform the control to such an extent that the position adjustment is completed within 10 seconds to 1 minute including changes in height and cast-piece thickness due to transverse movement. In addition, it is preferable that a liftable and lowerable range covers a nominal cast-piece thickness to be cast and can be controlled to about - 20 mm to +50 mm of the nominal cast-piece thickness.

The booms 11 and 12 need to maintain sufficient straightness and rigidity in transverse movement. Specifically, since the cast-piece 1 usually has a width of about 1 m to 2.5 m, it is necessary to be able to perform traveling measurement over the full width of the measurement target. In the case of the full-width measurement, it is necessary to be able to travel to the innermost part of the cast-piece 1. Note that on the assumption of left-right symmetry, it is also conceivable to design the boom so as to be able to travel to the center as a half-width measurement, and it is possible to design the boom according to the required accuracy, the degree of segregation, the customer's request, or the like.

A gap between casting segments is usually about 30 mm to 50 mm, and the boom needs to be inserted into the gap. Therefore, the thicknesses of the booms 11 and 12 in the casting direction are very thin. On the other hand, in order to cope with a cast-piece of 2 m in width and considering the retraction, it may be necessary to have a stroke of about 3 m to 4 m in the X direction, and it is necessary to have sufficient rigidity in order to prevent deformation or the like due to its own weight, the weight of a head, interference and rubbing with the cast segment, and the like. Therefore, it is necessary to have rigidity in the height direction.

The booms 11 and 12 receive radiant heat from the cast-piece 1. At this time, the way of heat entering is different between a surface facing the cast-piece 1 and the opposite side of the surface. A temperature difference occurs between the surface facing the cast-piece 1 (a boom lower surface in the boom 11) and the opposite side, and the warp of the boom occurs. Since the electromagnetic ultrasonic sensor is on the side away from the measurement target, this acts in a direction in which the lift-off becomes large, separately from a fluctuation in the thickness of the cast-piece 1. When the lift-off increases, the sensitivity decreases according to a lift-off characteristic of the electromagnetic ultrasonic sensor.

In addition, considering a thermal influence and maintainability at the time of non-measurement, it is desirable that the boom can retract to a position sufficiently away from the cast-piece 1, and more specifically, the boom preferably retracts to the outside of the casting segment or to the outside of a casting chamber. When the boom can retract to the outside of the casting chamber, it is possible to stand by without being affected by heat, steam, or the like, and maintenance can be performed at an arbitrary timing.

In addition, since the roll gap is about 30 mm to 70 mm as described above, the sensor, the boom, and the gap measuring unit need to have the size which can be inserted into this gap. Further, considering maintenance, it is desirable that the boom can be pulled out to the outside of the continuous casting facility. In addition, in the case of cantilever, when the tip end is lowered by the weight at the time of insertion, the electromagnetic ultrasonic sensor on the upper side comes into contact with the cast-piece 1, or the lift-off becomes too large in the electromagnetic ultrasonic sensor on the lower side, and the sensitivity cannot be maintained, so that the booms 11 and 12 preferably have sufficient rigidity. On the other hand, the double-sided holding is advantageous for these problems. On the other hand, when there is no margin in the layout of the continuous casting facility, it is difficult to realize the double-sided holding. In any case, it may be appropriately determined in consideration of the condition of the facility and the rigidity of the boom.

<Measurement Control Processing>

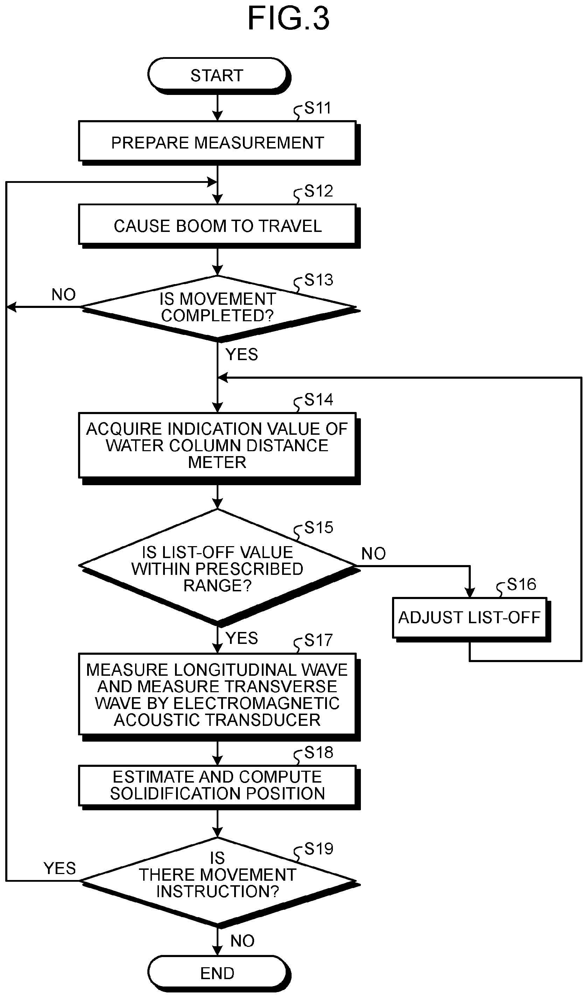

FIG. 3 is a flowchart illustrating a procedure of measurement control processing of the solidification position by the solidification position measuring apparatus 10. As illustrated in FIG. 3, first, when casting is started, the signal of start casting is transmitted from the host device 110 to the drive control unit 13 and the measurement computing unit 14. When the signal of start casting is detected, measurement preparation is performed (step S11). In this measurement preparation, measurement conditions (measurement pitch in the width direction, etc.) are determined based on a cast-piece thickness, a casting width, a steel type, and the like. In addition, at this time, the flow rate check of various kinds of cooling water and the soundness check of devices may be performed. Then, the boom height at the time of insertion is determined and adjusted based on the cast-piece thickness. A set casting thickness or a cast segment opening degree may be used as a reference. Specifically, the cast-piece thickness + 7 mm or the like can be exemplified, and here, a height to the extent that the cast-piece 1 is not contacted at the time of initial insertion is roughly adjusted. Note that it is desirable to set a control sequence in which abnormality notification and measurement stop are performed as lifting and lowering abnormality when adjustment cannot be performed to the set height.

Thereafter, the booms 11 and 12 are caused to travel transversely to the measurement position (step S12). With the completion of an initial height adjustment step in step S11, it is determined that a target cast-piece has reached a designated position based on an operating performance index such as a pull-out length, and the booms are moved by transverse traveling. In an initial stage of continuous casting, the sensor may be damaged due to running of a dummy bar, variations in initial cast-piece thickness, and the like. Therefore, completion of passing of the dummy bar or passing of the top of the cast-piece may be used as a trigger for the start of transverse movement. The first measurement standby position is a position where only the gap measuring unit at the tip end of the boom nears the edge of the cast-piece. Since the casting width is transmitted in advance as operating performance information and the position of the gap measuring unit is known, the position of a movement destination can be automatically calculated.

Thereafter, it is determined whether or not the movement of the booms 11 and 12 to the measurement standby position is completed (step S13). When the movement is not completed (step S13: No), the processing proceeds to step S12, and when the movement is completed (step S13: Yes), an indication value (detection value) of the water column distance meter 6 is acquired (step S14). Then, based on a lift-off value L2 measured by the water column distance meter 6, it is determined whether or not a lift-off value L1 between the cast-piece 1 and the electromagnetic ultrasonic sensor 7 is within a prescribed range (step S15). When the lift-off value L1 is not within the prescribed range (step S15: No), the lift-off value is adjusted (step S16), and the processing proceeds to step S14. The adjustment of the lift-off value L1 is performed by the lifting and lowering control of the boom 11.

The lift-off value may have an error due to the influence of the temperature, the casting speed, or the like, the displacement of the cast segment, the thermal expansion of the boom, or the like with respect to the casting thickness based on the nominal value/set value, and thus, the height of the electromagnetic ultrasonic sensor is adjusted by the actually measured gap. As the gap between the cast-piece and the electromagnetic ultrasonic sensor, for example, 5 mm $\pm$ 0.5 mm and the like can be exemplified. When the gap is large, the sensitivity decreases and measurement becomes impossible. On the other hand, when the gap is too small, the risks of contact, burning, and damage increase due to a fluctuation in the cast-piece thickness or the like.

When the lift-off value is within the prescribed range (step S15: Yes), longitudinal wave measurement and transverse wave measurement by the electromagnetic ultrasonic sensors 7 and 8 are performed (step S17). That is, a pulse current is applied to the electromagnetic ultrasonic sensor 8 to induce ultrasonic waves on the surface of the cast-piece. Then, the ultrasonic waves are received by the electromagnetic ultrasonic sensor 7, appropriate signal processing is performed, and solidification position estimation computation for calculating a solidification state and/or a solidification position (step S18) is performed. The computation result is sent to the host device 110. Note that depending on operating conditions and the like, only the longitudinal wave measurement or only the transverse wave measurement may be performed.

Thereafter, it is determined whether or not there is a movement instruction (step S19). When there is the movement instruction (step S19: Yes), the processing proceeds to step S12, and the booms are moved to the next measurement point. On the other hand, when there is no movement instruction (step S19: No), this processing is ended, and the electromagnetic ultrasonic sensors 7 and 8 and the like are moved to the outside of the continuous casting facility.

The movement instruction is set in advance, for example, to measure at a pitch of 100 mm in the width direction. By the movement of the electromagnetic ultrasonic sensors 7 and 8, zigzag measurement points are discretely obtained in the width direction with respect to the cast-piece 1, and the shape of one solidification position is obtained as a distribution in the width direction by performing the full-width measurement. The order of traveling may also be appropriately determined, for example, whether the measurement is performed in order from the edge of the cast-piece, or whether the measurement is performed from the central portion.

Note that a measurement processing end determination based on whether or not there is the movement instruction may be the end of casting. Information on the end of casting or the fact that a cast-piece top (upper end and tail end) has passed the designated position may be used as a trigger for the end of measurement. In particular, the boom is desirably retracted because unsolidified molten steel may be exposed at the cast-piece top or the cast-piece thickness may fluctuate due to disappearing of a static iron pressure or solidification shrinkage. When the boom is retracted, the boom may be retracted at the height at that time. However, for example, by retracting the boom by lifting the boom by about 2 mm to 3 mm (in a direction in which a distance from the cast-piece increases), the boom can be retracted without risks such as a deviation in the cast-piece width direction and a boom inclination. The retraction to the outside of the continuous casting facility is completed, and the measurement is ended.

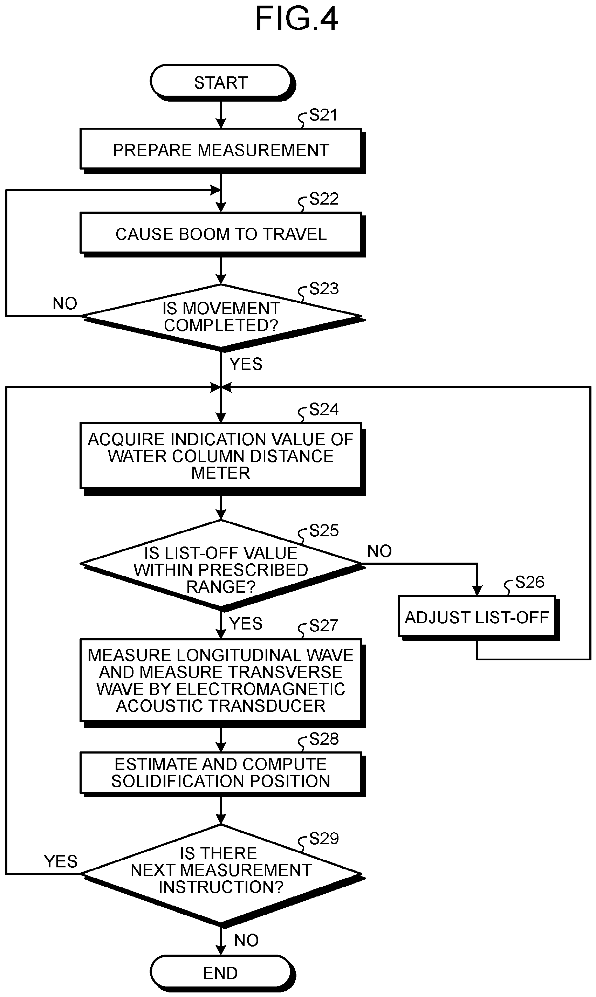

Note that it is also conceivable that measurement is continuously performed at a specific position (specific position measurement). In this case, there is no movement instruction for movement of the measurement point, and the measurement is performed according to the procedure illustrated in the flowchart of FIG. 4. In the specific position measurement, it is conceivable to intensively measure the center position of the cast-piece or a position particularly affecting the quality of the cast-piece from the empirical/past results, and the specific position is appropriately determined according to the properties and requirements of the target cast-piece.

As illustrated in FIG. 4, in the specific position measurement, the same processing as the processing in steps S11 to S18 is performed (steps S21 to S28). In step S28, after the solidification position estimation computation is performed, it is determined whether or not there is a next measurement instruction (step S29). In the specific position measurement, it is not necessary to cause the boom to travel. Therefore, when there is the next measurement instruction (step S29: Yes), the processing does not proceed to step S22 but proceeds to step S24 to acquire the value of the water column distance meter and perform measurement at the same position. On the other hand, when there is no next measurement instruction due to the end of casting or the like (step S29: No), this processing is performed.

<Arrangement Modifications of Boom, Electromagnetic Ultrasonic Sensor, and Water Column Distance Meter>

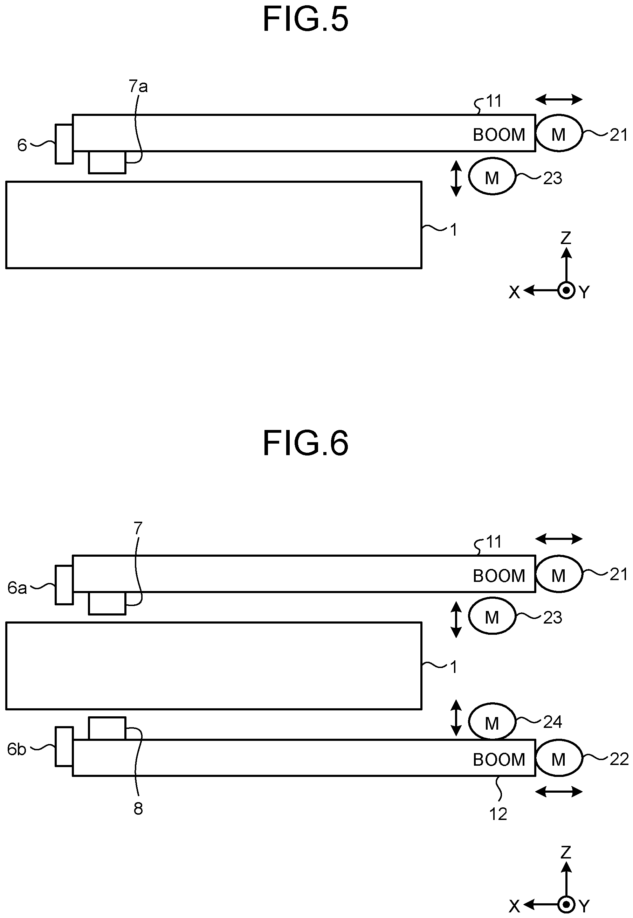

FIGS. 5 and 6 are diagrams illustrating examples of arrangement modifications of the boom, the electromagnetic ultrasonic sensor, and the water column distance meter. FIG. 2 illustrates a transmission measurement system in which the electromagnetic ultrasonic sensors 7 and 8 are disposed so as to sandwich the cast-piece 1 as a measurement target, but as illustrated in FIG. 5, a reflection measurement system using one electromagnetic ultrasonic sensor 7a may be used. In the case of the reflection measurement system, the boom 12 and the motor 22 are not provided. Note that the electromagnetic ultrasonic sensor 7a may be provided on the lower side of the cast-piece 1.

In addition, as illustrated in FIG. 6, water column distance meters 6a and 6b may be provided for both of the booms 11 and 12, and a motor 24 as the sensor lifting and lowering unit may be further provided for the boom 12. The transmission measurement system has a shorter propagation distance of ultrasonic waves than the reflection measurement system, and thus has an advantage that signal intensity is easily obtained. Note that as illustrated in FIG. 2, the continuous casting facility has the mechanism in which the casting thickness is controlled by controlling the position of the roll on the upper side basically with the lower side as a reference surface in many cases. In this case, it is preferable to have a structure in which the sensor height on the lower side is fixed and the height on the upper side is controlled.

<Water Column Distance Meter>

In the case of an optical method such as a laser distance meter are used as the gap measuring unit, a commercially available and general red laser light source is used for a measurement target that is a red-hot cast-piece. However, the red laser light source cannot be used appropriately in many cases. In addition, it is often difficult to use the red laser light source in the vicinity of the cast-piece due to interference of a laser beam for measurement by water droplets, mist, oil mist, or the like accompanying cooling of the cast-piece, contamination of an optical window, or the like.

In addition, a eddy current type distance meter is characterized by high accuracy and relatively compact. On the other hand, since a distance to a measurement target is limited to several millimeters or less, the risk of wear and failure due to a thermal influence tends to increase. Then, because of the principle of measuring a distance to a conductor, the exterior of the sensor is mostly made of plastic or ceramics. Here, the plastic may be a problem in terms of heat resistance, and the ceramic may be a problem in terms of mechanical strength due to thermal load or the like.

On the other hand, the water column distance meter is most suitable as the gap measuring unit of the solidification position measuring apparatus according to the present invention. Some reasons will be mentioned. First, due to the structure in which ultrasonic waves are propagated by a water column, the water column distance meter is hardly affected by water vapor, mist, or the like. Then, since reflection occurs due to a difference in acoustic impedance between the surface of the cast-piece and water, measurement can be performed regardless of the temperature of the surface of the cast-piece. Even when water in contact with the surface of the cast-piece generates bubbles due to boiling, the bubbles are immediately flowed by the water pressure of the water column, and reflection occurs from the surface of the cast-piece. In addition, since an ultrasonic probe has a structure that is always water-cooled, wear and failure due to a thermal influence hardly occur. For the length of the water column, a distance of about 50 mm can be taken as described later, and a thermal influence on a nozzle and the like is also small. In addition, the water column distance meter itself is small, and can be used in a narrow place such as a gap between segment rolls. The water column distance meter has a total thickness of about 20 mm to 30 mm, and can be sufficiently inserted in the gap between the rolls.

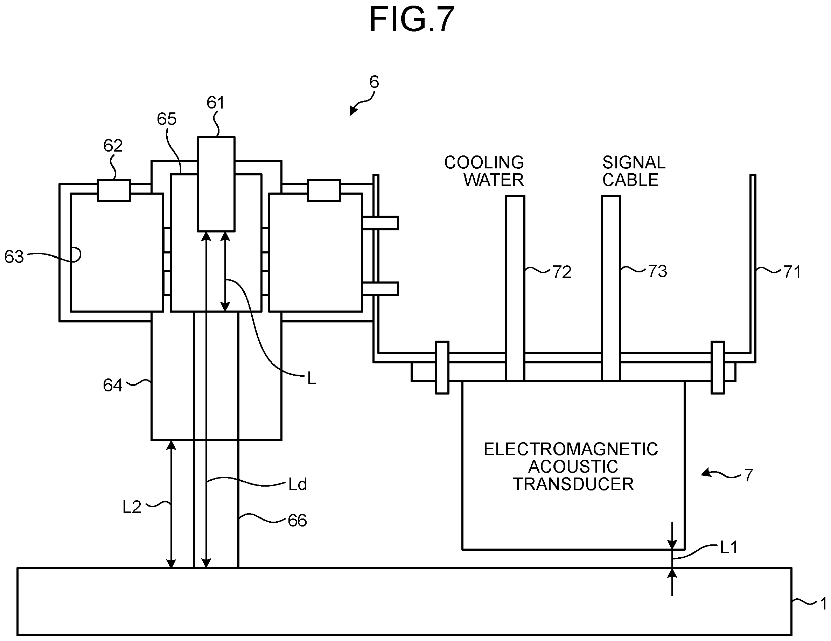

FIG. 7 is a cross-sectional view illustrating a configuration of the water column distance meter 6. The water column distance meter 6 typically has a structure as illustrated in FIG. 7, but is not limited thereto. The water column distance meter 6 includes an ultrasonic probe 61, a water supply port 62, a water tank 63 for straightening, a nozzle 64, and a step 65.

The ultrasonic probe 61 is preferably a local water immersion probe. A probe that can be used for submersion may be selected for mounting in water for a water column. The water for the water column also serves as cooling water for the probe. A frequency and probe dimensions may be appropriately designed, but a probe having a center frequency of 5 MHz can be exemplified. As the diameter of the probe, about 8 mm to 12 mm can be exemplified.

An appropriate amount of water is supplied to the water supply port 62, and a water column is formed to a target through the nozzle 64. The amount of water to be supplied depends on a nozzle diameter, a water distance, and particularly a distance from a tip end of the nozzle 64 to the cast-piece 1, and thus may be appropriately designed. As an example, when the nozzle is of 8 mm and the distance between the tip end of the nozzle and the cast-piece 1 is 50 mm, the amount of water is preferably about 5 to 15 liters/minute.

With respect to the quality of the water to be supplied, it is desirable that air bubbles, dust, and the like are not included. Air bubbles and the like in the water column serve as a reflection source of ultrasonic waves, and cause a decrease in an S/N ratio (Signal to Noise Ratio) or an entire failure in obtaining a reflection signal. However, some air bubbles may be removed by processing such as synchronous addition or averaging.

In addition, a straightening/retention mechanism can be provided in the water column nozzle to take measures against air bubbles. By designing the water column nozzle so as to prevent the retention of air bubbles in the vicinity of a sensor surface, it is possible to reduce the influence of air bubbles that may be included to some extent.

The nozzle diameter is preferably about 6 mm to 15 mm, and more preferably 8 to 10 mm. The flow rate is preferably about 5 to 15 liters/minute, and the flow rate and a nozzle distance are designed so as to generate a laminar flow. The nozzle distance (water column distance) may be appropriately designed from the nozzle diameter and the water column flow rate, and a range of 5 mm to 100 mm can be exemplified. Typically, the nozzle distance is preferably about 30 mm to 70 mm. When the nozzle distance is about 5 mm or less, a problem of contact with the cast-piece 1 or a thermal influence on the ultrasonic probe 61 occurs. When the distance of the water column increases, it is necessary to increase the flow rate in order to maintain a stable water column, but the increase in flow rate causes turbulence and the S/N ratio of the reflection signal decreases. In addition, it is necessary to increase the flow rate or increase a water pressure, both of which cause turbulence.

In addition, the nozzle diameter may be selected to be about the same as the probe diameter. When the diameter is small, the intensity of ultrasonic waves that can be propagated is reduced, and sensitivity and stability are deteriorated. By setting the nozzle diameter to be about the same as the probe diameter, a part of an ultrasonic signal propagating in a slightly spreading manner is reflected by the step 65 and can be used as a reference echo. When the nozzle diameter becomes too large, a step echo becomes too small, or it is necessary to increase the flow rate, so that unnecessary cooling may be applied to the cast-piece 1, which is undesirable.

Further, by performing straightening, flow velocity adjustment, and degassing of air bubbles in the water tank 63, the retention of air bubbles is not caused on a front surface of the ultrasonic probe 61 or the like, scattering of ultrasonic waves can be prevented, the S/N ratio can be improved, and measurement can be stabilized. As illustrated in FIG. 7, the water tank may be provided on both sides or on one side of the ultrasonic probe 61. It may be appropriately designed from installable dimensions or the like.

The step 65 is provided to partially reflect the ultrasonic wave by the step 65 and correct the temperature dependency of a sound velocity by a reflection signal of the ultrasonic wave. A boundary between a space for storing the ultrasonic probe 61 and the nozzle 64 may be the step 65, or the step may be formed by reducing the diameter by the nozzle 64. In addition, a protrusion or a rod shape may be provided so as to cause partial reflection.

When the ultrasonic wave reflected by the step 65 returns to the ultrasonic probe 61, an echo (step echo) is generated. Water temperature correction is performed using this step echo. Since the ultrasonic sound velocity changes depending on water temperature, a distance Ld cannot be calculated only by an echo from the cast-piece 1. On the other hand, the distance can be obtained with high accuracy by using the step echo.

Here, the distance to the cast-piece 1 is Ld. When a measured propagation time is Td, the sound velocity is V(C), and the water temperature is C, the distance Ld desired to be obtained can be expressed as Ld = Td/V(C). Since the sound velocity has dependency on the water temperature, an error caused by V(C) (temperature dependence) occurs. In order to prevent this error, it is necessary to hold a sound velocity table in advance, measure the water temperature, refer to the table, and calculate the water distance by the sound velocity according to the temperature, which becomes complicated as a system and calculation.

On the other hand, in the distance measurement using the step 65 of the embodiment, a distance from the ultrasonic probe 61 to the step 65 is L, and the distance from the ultrasonic probe 61 to the cast-piece 1 is Ld. In addition, a propagation time of the echo from the step 65 is T, and a propagation time of the echo from the cast-piece 1 is Td. The distance from the ultrasonic probe 61 to the step 65 is a known value determined by machining accuracy, and is substantially a constant. The distance Ld can be obtained by the following equation using the propagation time T of the step echo and the distance L to the step 65.

$$Ld = L/T \times Td$$

As is clear from this equation, the distance Ld can be expressed in a form not including a temperature-affected term, and distance measurement can be performed without depending on the water temperature. The propagation time of the step echo and the propagation time from the cast-piece 1 are obtained with the same degree of accuracy, and the influence of the temperature dependency of the sound velocity is eliminated.

It is possible to perform real-time water temperature correction by setting detection gates of ultrasonic waves for two of the step echo and the echo from the cast-piece 1 and simultaneously acquiring the echoes. Therefore, it is preferable to process captured waveforms using a personal computer or the like and perform distance measurement.

Note that in a sound wave measuring device and a temperature correction method thereof described in Patent Literature 2, temperature correction is performed by obtaining an echo propagation time from a step in advance for temperature correction and then measuring an echo propagation time from a measurement target. On the other hand, the embodiment is different in that temperature correction is performed by simultaneously acquiring the step echo and the echo from the cast-piece 1 using the fact that partial reflection from the step always occurs. By simultaneously acquiring the echoes, measurement with high accuracy and to which constant temperature correction is applied becomes possible when the water temperature changes, for example. The propagation time and the time of the step echo are simultaneously measured, and thus, as a result, accuracy becomes higher than that of a premeasurement method.

In addition, by the distance measuring method using the water column distance meter 6 of the embodiment, it has also been confirmed that an absolute value error is within 0.1 mm over a distance range of 20 mm to 50 mm from the nozzle 64 to the cast-piece 1.

<Positional Relationship between Water Column Distance Meter and Electromagnetic Ultrasonic Sensor>

The water column distance meter 6 is installed ahead of the electromagnetic ultrasonic sensor 7, the distance between the cast-piece 1 and the electromagnetic ultrasonic sensor is constantly measured, and the water column distance meter and the electromagnetic ultrasonic sensor can be disposed as illustrated in FIG. 7.

As illustrated in FIG. 7, since the electromagnetic ultrasonic sensor 7 and the water column distance meter 6 are mechanically coupled to each other, the relative position may be grasped in advance. Typically, a difference between a tip end surface of the electromagnetic ultrasonic sensor 7 and a tip end position of the nozzle 64 is about 30 mm to 50 mm. It may be appropriately set according to stability of a water column 66 or restrictions on an installation structure.

<Electromagnetic Ultrasonic Sensor>

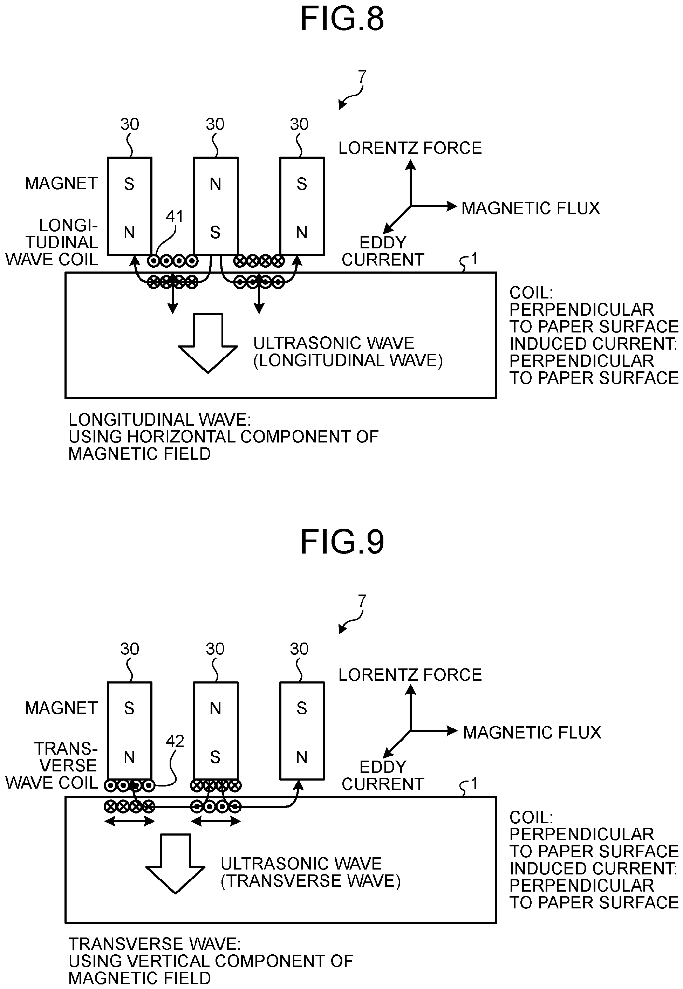

An electromagnetic ultrasonic wave is an ultrasonic vibration induced in a conductor by a Lorentz force due to an interaction of an eddy current and a static magnetic field. The eddy current is induced in the conductor by a large current pulse signal applied to a coil and flows along a conductor surface. Since the Lorentz force is generated according to Fleming's left-hand rule, a longitudinal wave can be generated when a magnetic field parallel to the conductor surface is applied as illustrated in FIG. 8, and a transverse wave can be generated when a magnetic field perpendicular to the conductor surface is applied as illustrated in FIG. 9.

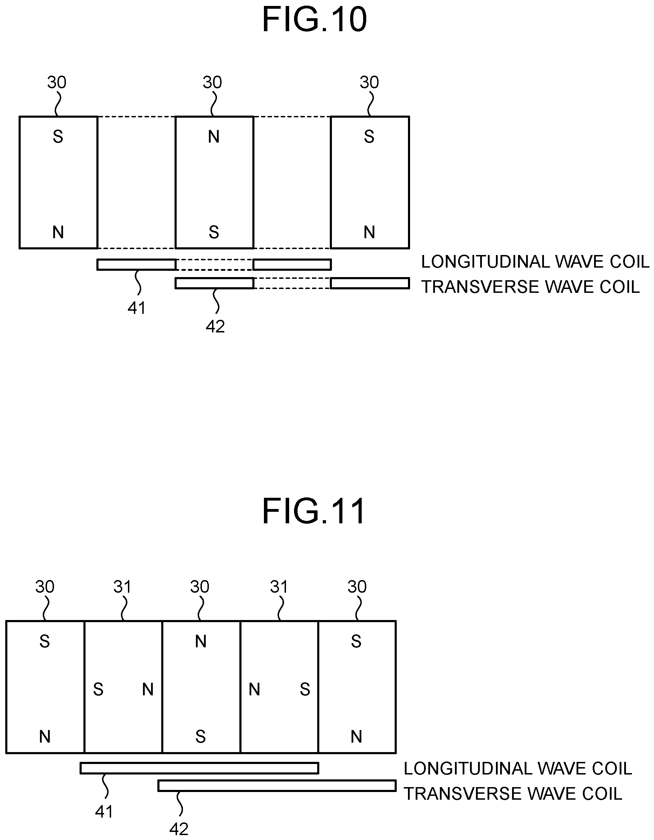

As illustrated in FIG. 10, the electromagnetic ultrasonic sensor can generate a longitudinal wave and a transverse wave by arranging three magnets 30, a longitudinal wave coil 41, and a transverse wave coil 42. In the magnets 30 of the electromagnetic ultrasonic sensor illustrated in FIG. 10, a set of magnets 30 can generate, for the cast-piece 1, a magnetic field for a transverse wave and a magnetic field for a longitudinal wave.

With respect to the cast-piece 1, the longitudinal wave coil 41 is placed in an area where the horizontal component of magnet field is dominant, and the transverse wave coil 42 is placed in an area where the vertical component of magnet field is dominant. Schematic diagrams of magnetic force lines are also illustrated between the magnetic poles. A longitudinal ultrasonic wave is generated on the surface of the cast-piece 1 by a magnetic flux of the horizontal component, and a transverse wave is generated by a magnetic flux of the vertical component. More specifically, in the longitudinal wave, according to Fleming's left-hand rule, the Lorentz force is generated in the vertical direction in the drawing by an interaction between the horizontal magnetic field and an eddy current induced on the surface (a direction perpendicular to the drawing surface) A compressional wave is generated with respect to a propagation direction of the ultrasonic wave, so that the longitudinal wave is generated. In the transverse wave, the Lorentz force using eddy current in the direction perpendicular to the drawing surface and vertical magnetic field generate generates a vibration of a direction perpendicular to a applied material is generated in the horizontal direction in the drawing, which results in a shear wave (transverse wave) with respect to the propagation direction.

By using the magnet with the size of each magnet to about 20 mm (casting width direction) $\times$ 30 mm (casting pulling-out direction) $\times$ 40 mm (height), it is possible to build a small sensor having a size of about 100 mm in the casting width direction and about 30 mm in the casting direction and capable of generating both the longitudinal wave and the transverse wave. Further, including a water path for cooling and a water-cooling jacket for holding the water path, the sensor can be a small electromagnetic ultrasonic sensor that can install to a casting segment (typically 200 mm$\varphi$ roll and roll gap 50 mm) .

In addition, a magnetic pole width also affects the number of windings of the coil and the wire width thereof. On the transmission side, when the magnetic pole width is narrowed, it is necessary to narrow the coil which causes adverse effects such as an increase the impedance (particularly, DC resistance) of the coil, an inability to withstand an applied current, and an inability to obtain an optimum number of windings. Also on the reception side, it is necessary to reduce the number of windings for detection, and sensitivity decreases.

Further, as illustrated in FIG. 11, Halbach array magnets may be used, which is obtained by adding an auxiliary magnet 31 whose magnetic pole direction is orthogonal to magnets By making magnetic pole intervals equal and adding the auxiliary magnet 31, it is also possible to enhance the magnetic field and improve sensitivity without changing magnet size. Specifically, instead of a spacer (nonmagnetic/nonconductive) in the arrangement of the magnets 30 illustrated in FIG. 10, the auxiliary magnet 31 having the same size is added. As a result, a magnetic field strength is improved by about 25%, and sensitivity can be improved. An improvement in measurement sensitivity is effective in performing automatic measurement. When a waveform is unclear (S/N is low), automatic determination cannot be performed, and determination is eventually suspended by manual analysis or missing.

Next, a pancake type or racetrack type coil corresponding to the magnetic pole interval is placed on a front surface of the magnet. A coil material may be appropriately designed, but a flat wounded enameled wire , or a patterned coil using a flexible printed circuit board are preferable. Using the flexible printed circuit board eliminates manual coil production, which provides advantages that characteristic fluctuations are reduced and the lift-off can be substantially reduced by reducing the total thickness. A wire diameter and the space of the coil are appropriately determined, but it is desirable that the thickness of copper foil is as thick as possible and the wire diameter is large on the transmission side so that a large current can be applied. Although the eddy current to be induced increases by increasing the number of turns by increasing the number of windings, there is a trade-off that an applicable current decreases due to an increase in impedance of the coil. The balance between them may be determined experimentally or by calculation. On the reception side, it is desirable to increase the number of windings as much as possible.

The coils and the magnets are placed in the water-cooling jacket. A main body of the water-cooling jacket is desirably made of SUS. By using a nonmagnetic material, it is possible to obtain both mechanical strength and water cooling efficiency without affecting a magnetic field distribution. Although a surface facing the cast-piece 1 is referred to as a front panel, it is necessary to design the front panel so as to pass a static magnetic field by the magnet and an electromagnetic field by a current pulse applied to the coil. Therefore, the front panel may be a nonmagnetic SUS plate having a thickness of about 0.2 mm or ceramics. In particular, a thin SUS plate has heat resistance, toughness, and also abrasion resistance, and is nonmagnetic, so that an electromagnetic field relatively easily passes therethrough, and a sensitivity influence is small. Ceramics are nonmagnetic and nonconductive, but have poor workability and poor strength in terms of toughness. In order to obtain a practical strength, it is necessary to have a thickness of 1 mm to 2 mm, and as a result, the lift-off becomes large. Therefore, it is preferable to use a thin nonmagnetic SUS plate.

The electromagnetic ultrasonic sensor configured as described above is brought close to a predetermined position with respect to a measurement target, a current pulse is applied to a transmission coil, and electromagnetic ultrasonic waves are generated. The generated ultrasonic waves propagate in the cast-piece 1 as the measurement target, and vibrate the surface (reception surface) of the cast-piece 1. On the reception surface, a current is induced on the surface by the propagated vibration and the static magnetic field by an electromagnet on the reception side, and a change in the surface current is detected as an electric signal by a reception coil. The received electric signal is amplified by a preamplifier having an appropriate band and gain, and is taken into a PC or the like.

<Chirp Pulse Compression and Signal Processing>

In transmission/reception processing of electromagnetic ultrasonic waves, a chirp pulse compression technique may be used. The chirp pulse compression is to use a signal having a waveform in which a frequency changes with time (generally, also referred to as a chirp signal or a linear frequency modulated signal. In this specification, the signal is referred to as the chirp signal) for transmission and to perform, on a waveform of a reception signal, computing processing including a correlation computation on the waveform of the receiving signal. By using the chirp pulse compression processing as the transmission/reception processing of the electromagnetic ultrasonic wave, it is possible to reduce noise and improve time resolution.

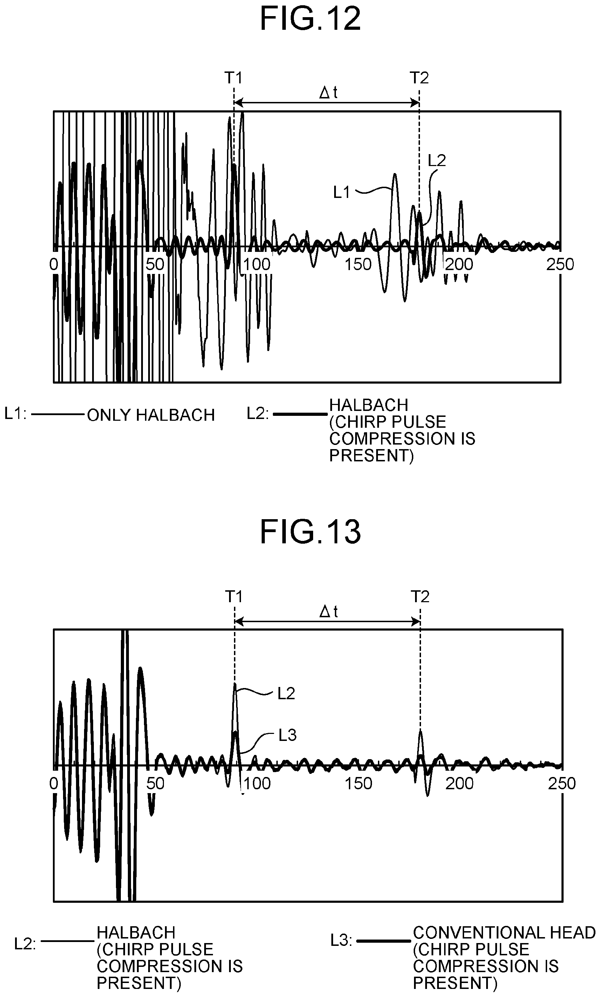

FIG. 12 is a diagram illustrating a reception signal waveform depending on the presence or absence of the chirp pulse compression with the magnets of the electromagnetic ultrasonic sensor arranged in a Halbach array. A waveform L1 is a waveform in a case where the chirp pulse compression is not performed, and a waveform L2 is a waveform in a case where the chirp pulse compression is performed.

In the embodiment, the waveform after the application of the chirp pulse compression (that is, the above computing processing) is further subjected to processing of waveform detection in a first gate (a time gate near the transmitted wave T1) and a second gate (a time gate near the 1.5 times of transmitted wave T2), and propagation time measurement based on the time difference.

In a conventional ultrasonic wave transmission/reception method, a transmission signal has a sensitivity as best as detect even using the chirp pulse compression. Therefore, in conventional measurement of a solidification position, performing electromagnetic ultrasonic measurement and it has been necessary to remove noise manually or extract and determine an appropriate waveform to calculate the ultrasonic propagation time, so that automatic measurement has been difficult.

The propagation time of the ultrasonic wave is physically defined by a time from transmission to reception of the ultrasonic wave. However, since a high frequency pulse of a large current is used for electromagnetic ultrasonic wave generation, large electromagnetic noise occurs at the moment of transmission and leaks into a reception signal. Therefore, it is difficult to determine a transmission origin, and it is difficult to calculate the propagation time only from the reception waveform.

For actual usage, it is necessary to determine a reference of the transmission time (transmission origin: T0), and the transmission origin has been determined by the following method, for example.

1) Determination by Offline Simulated Sample

Ultrasonic waves are transmitted and received using a sample having a known plate thickness and a known sound velocity. Then, the transmission origin can be calculated from the position of a reception waveform. However, if there is an error in the sound velocity or the like, all of them may shift.

2) Online Measurement using Thin Slab

The transmission origin may be calibrated by measurement using a thin slab. When the slab is thin, the attenuation is small, and thus multiple reflection waveforms are easily detected. When a once-transmitted wave is a T1 wave and a 1.5-reciprocating transmitted waveform reflected on each surface of the slab is a T2 wave, the transmission origin T0 can be calculated by T0 = T1 - (T2 - T1) a 2 using, for example, the time T1 of a peak of the T1 wave and the time T2 of a peak of the T2 wave. Hereinafter, a case where the time is calculated using a peak position of the waveform will be described, but the time can be calculated with substantially the same concept even when zero crossing, envelope processing, or the like is performed.

In addition, the sensitivity can be improved by performing measurement by bringing the sensor closer for a short time taking a contact risk into consideration, and the transmission origin can be similarly calibrated as a condition under which the T2 wave is output. It is known that the sensitivity is usually improved about 6 dB when the sensor is closer about 1 mm each. However, there are risks of damage due to contact and the damage and burning of the sensor due to an increase in thermal load, and it is naturally difficult to operate the sensor at all times.

When the transmission origin T0 is determined in this way, a propagation time $\Delta t$ can be calculated as $\Delta t = T1 - T0$ in normal measurement. However, there have still been problems in the propagation time calculation method with a fixed transmission origin.

First, the transmission origin may change due to individual differences and frequency characteristics of pulsers, variations in frequency characteristics of coils, and the like. Since the transmission origin also may change with aging, it is necessary to periodically check and recalibrate the transmission origin. When the transmission origin changes, an offset occurs in the propagation time, so that erroneous determination occurs in the measurement of the solidification position.

In addition, when a surface temperature of the slab changes, particularly under a condition where the temperature falls below the Curie point due to supercooling, the waveform of an electromagnetic ultrasonic wave to be transmitted and received may be distorted. At this time, a phase changes, and an apparent propagation time changes. Naturally, in the case of performing peak detection, the propagation time changes with a change in phase, and an error occurs in the measurement.

Normally, when an electromagnetic ultrasonic wave is used to measure the solidification position, an ultrasonic wave at a low frequency of several tens kHz to several 100 kHz (assumed to be lower than 500 kHz) is used. A change in peak position due to the change in phase at this time is a change of about 1/2 waveform of a main frequency, and thus, in the case of 100 kHz, the change in peak position is corresponded several $\mu$s. This is several times larger than the change in propagation time to be usually measured, and thus becomes an abnormal value.

Note that in ultrasonic flaw detection, zero crossing can be used as signal detection. However, in the measurement of the solidification position using the electromagnetic ultrasonic wave in which the accuracy of the propagation time is important, it is more preferable to use the peak detection described above.

Therefore, in the embodiment, the sensitivity of transmission/reception is greatly improved by adopting the Halbach array, and thus, it is possible to measure the propagation time without variations even with the change in transmission origin or in a state of changes in waveform and phase by using single reflection (1.5-reciprocating waveform in transmission measurement).

The time of a direct transmitted wave is T1, the time of a wave which reflected once on upper surface and once on lower surfaces is T2, and the propagation time $\Delta t$ is calculated as $\Delta t = (T2 - T1) \div 2$. According to this processing, even when the transmission origin fluctuates, the propagation time $\Delta t$ can be calculated as a time difference between the time T1(transmitted wave) and the time T2(the 1.5-time reciprocating wave). In addition, even when waveform distortion occurs, a waveform to be detected has a similar shape. This is because T2 is smaller due to attenuation of propagation. As a result, each peak may be detected to calculate the propagation time $\Delta t$.

Here, in conventional magnets used for electromagnetic ultrasonic wave generation, the magnets have been simply arranged with low sensitivity, so that it has not been possible to always observe the T2 waveform. FIG. 13 is a diagram illustrating a reception waveform (waveform L2) in a case where a sensor head with Halbach array magnets and the chirp pulse compression processing are used in combination, and a reception waveform (waveform L3) in a case where a sensor head with the normal magnets illustrated in FIG. 10 and the chirp pulse compression processing are used in combination.

In FIG. 13, the position of the time T1 can be confirmed for both the waveforms L2 and L3. However, the position of the time T2 can be confirmed for the waveform L2 using the Halbach array magnets, but cannot be confirmed for the waveform L3. For this reason, when the Halbach array is not used, it is necessary to define the transmission origin in advance, but the transmission origin may change as described above, and the propagation time cannot be obtained with high accuracy in the conventional method.

On the other hand, when the chirp pulse compression processing and the Halbach array magnets are used, the times T1 and T2 can be discriminated, and the propagation time $\Delta t$ can be obtained by $\Delta t = (T2 - T1) \div 2$. Note that it is conceivable to perform envelope processing on the phase change, but since the envelope processing corresponds to frequency reduction, time resolution deteriorates.

In addition, supercooling of a surface may occur in slabs of continuous casting. This supercooling occurs due to nozzle clogging, failure of a cooling spray, or local uneven flow due to falling off of a spary tip. In addition, the supercooling may also occur by flowing of cooling water through a division area (a chock of a division roll) of a casting roll of continuous casting.

Note that in a case where measurement is performed in a situation where there is local supercooling, a unimodal peak can be confirmed in a portion where there is no supercooling, and when the transmission origin is determined in advance, the propagation time can be obtained by the transmission origin and a peak position at the time T1. On the other hand, a waveform is different in a supercooled area, and specifically, the waveform does not have a unimodal peak and may have substantially the same amplitude on the plus side and the minus side. In the case of acquisition at the peak position, a peak on a latter side of the waveform is detected, so that the propagation time is calculated to be long.

On the other hand, in the embodiment, the peaks of the T1 wave and the T2 wave are picked up, and the time difference is obtained. Since the waveform of multiple reflection has a similar shape, it is sufficient to gate each waveform appearance position and perform maximum value detection or the like. As a result, the propagation time can be automatically calculated even in a region where the phase changes, such as the supercooled portion.

As described above, by improving the transmission/reception sensitivity using the Halbach array, calculation of the propagation time can be automatically performed even for a measurement target having temperature unevenness and variations in surface magnetic characteristics such as an actual cast slab.

Note that in a case where an ultrasonic signal is transmitted and received using a single ultrasonic sensor, the time of once-reflected wave reception may be T1, and the time of twice-reflected wave reception may be T2.

By using the Halbach array and the propagation time calculation procedure by the signal processing in combination with the transverse movement mechanism and the lifting and lowering control mechanism described above, automation of electromagnetic ultrasonic measurement and automation of solidification measurement in a continuous casting machine are achieved.

<Transverse Wave Measurement>

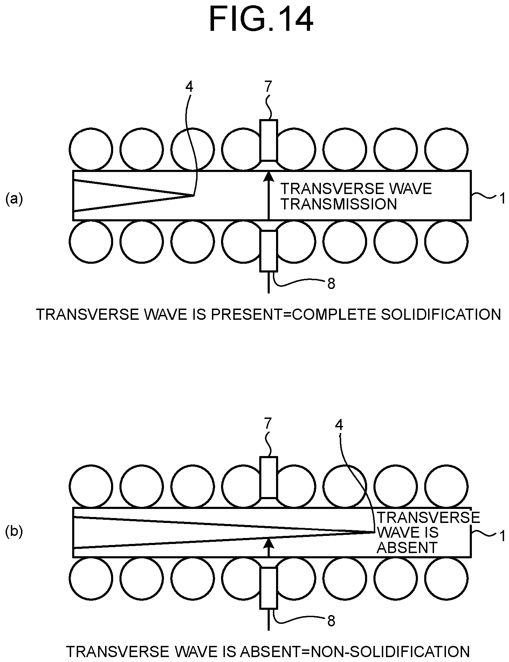

FIG. 14 is an explanatory diagram for explaining transverse wave measurement. In the case of the transverse wave measurement, since the transverse wave does not pass through the unsolidified liquid phase portion, the transverse wave can be received on the reception side when the solidification point 4 has not reached. When the solidification point 4 has reached, the transverse wave cannot be received on the reception side. Therefore, when the transverse wave is received, the cast-piece 1 is completely solidified, and when the transverse wave is not received, it can be determined that the cast-piece 1 is unsolidified.

<Longitudinal Wave Measurement>

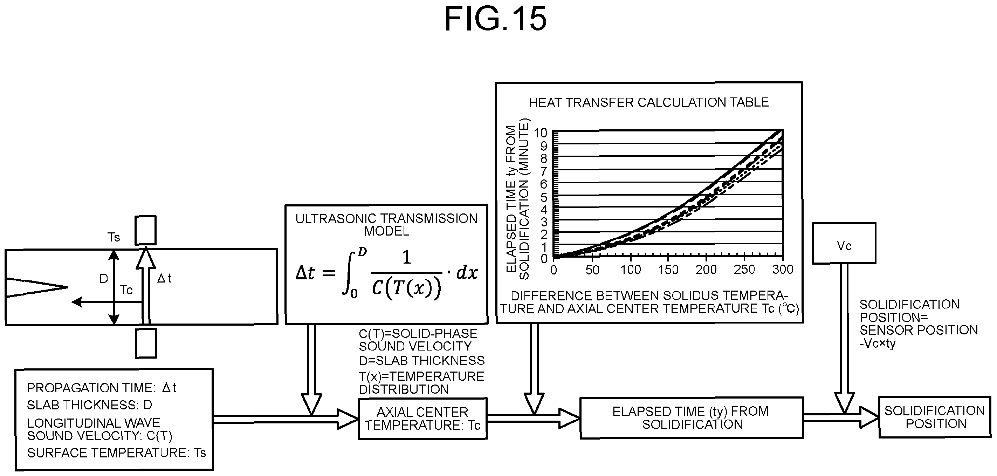

FIG. 15 is an explanatory diagram for explaining longitudinal wave measurement. In the case of the longitudinal wave, the ultrasonic wave propagates regardless of the presence or absence of the liquid phase portion. An center temperature Tc is obtained based on the propagation time $\Delta t$ of the longitudinal wave, values such as a thickness D of the cast-piece 1, a longitudinal wave sound velocity C(T), and a surface temperature Ts, and an ultrasonic transmission model. Then, an elapsed time ty from solidification is obtained based on a heat transfer calculation table for each characteristic of the cast-piece obtained in advance, and the solidification position 4 is estimated by the following equation using a casting speed Vc of the cast-piece 1.

Solidification position = position of electromagnetic ultrasonic sensor - Vc $\times$ ty

Note that in the heat transfer calculation table, a relationship between information on the center temperature Tc and the elapsed time ty from solidification is obtained. The heat transfer calculation table is, for example, a database of heat transfer calculation results based on operating conditions such as a steel type to be measured and a cooling condition. In the case of a graph, as in the example in FIG. 15, the horizontal axis represents a "difference between a solidus temperature and the center temperature Tc (unit: °C)", the vertical axis represents the "elapsed time ty from solidification (unit: minute)", and a plurality of plots reflecting a difference in the operating conditions are obtained. When the solidification position is measured, an appropriate plot (also possible in the form of a relational expression) is selected for each operating condition. In addition, the operating conditions such as a pouring rate and the amount of cooling water may be changed, the longitudinal wave propagation time under the conditions may be directly compared with the solidification position calculated by heat transfer calculation or the like, and a coefficient may be calibrated as an empirical formula. When the measured longitudinal wave propagation time is substituted into the empirical formula, the solidification position can be calculated.

<Automatic Measurement in Width Direction>

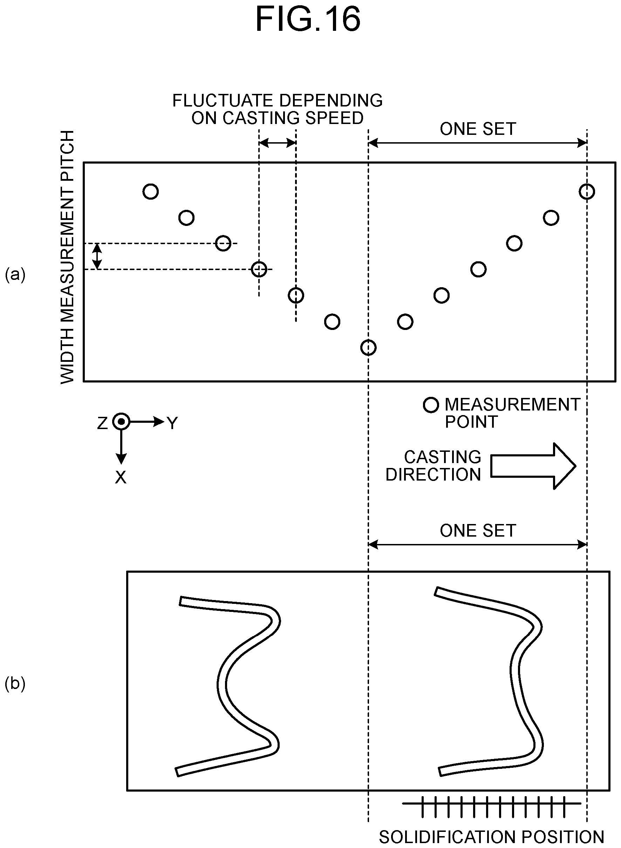

When one lift-off adjustment and electromagnetic ultrasonic measurement are completed, it is possible to perform automatic measurement in the full width of the cast-piece 1 by giving an instruction to move to the next measurement position. In order to perform the measurement while moving transversely the electromagnetic ultrasonic sensor in the width direction, the electromagnetic ultrasonic sensor is moved transversely according to a predetermined measurement pitch in the width direction as illustrated in FIG. 16, and the measurement is performed at that position. Since the cast-piece 1 moves in the casting direction, the measurement points form a zigzag when the electromagnetic ultrasonic sensor moves in the width direction of the cast-piece 1. When the full-width measurement in one direction is completed, the data is used as one set and a solidification position shape of each set can be calculated (see FIG. 16(b)).

<Automatic Measurement in Width Direction>

The measured solidification position and solidification position shape are made to correspond to a cast-piece (slab: a semi-finished product), and pass/fail determination of the slab is performed. As described above, the measurement is performed in the width direction, and the solidification position is calculated as a distribution in the width direction. The solidification position and solidification position shape are associated to each slabs. Then, one set of solidification position and solidification shape data in the width direction is associated with one slab, and the pass/fail determination is performed. Usually, each slab and each set of date do not match, so the pass/fail determination for one slab is performed based on the pass/fail determination result of one or more sets of data.

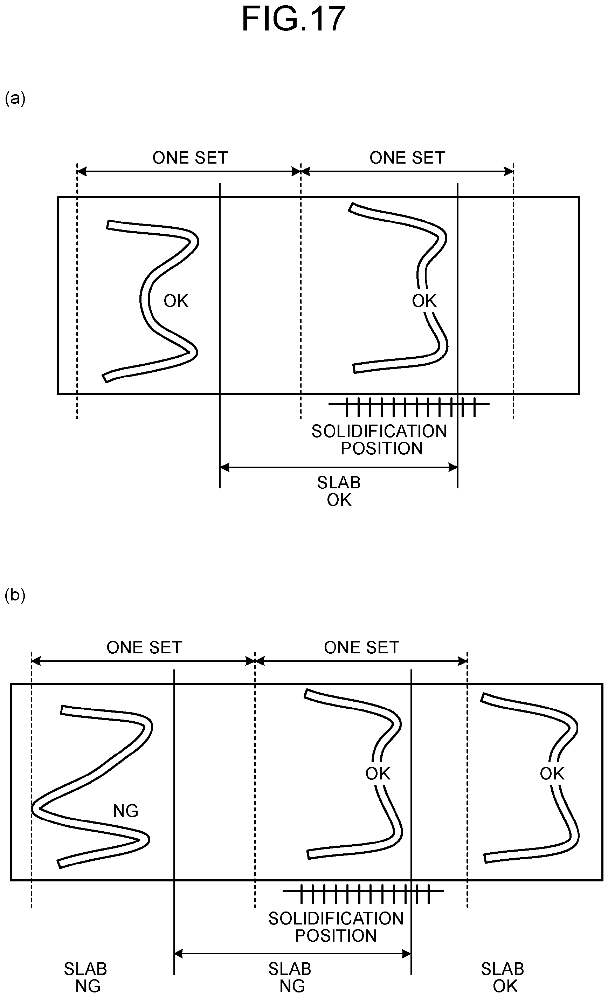

In FIG. 17(a), one slab is determined to be a pass (OK) because each of two sets associated with one slab is a pass (OK). On the other hand, in FIG. 17(b), since one of two sets associated with one slab is a pass (OK) but the other is a fail (NG), one slab is determined as a fail (NG).

Note that specifically, for the pass/fail determination of each set, the pass/fail determination is performed based on whether or not the solidification position deviates from a preset range, whether or not the solidification position shape is included in a designated range, and the like.

In the determination of the solidification position, for example, when molten steel is solidified out of a soft reduction zone, it is inappropriate because soft reduction is not appropriately performed. In addition, when only a part of the molten steel is in a state where solidification is delayed in the width direction, an additive component contained in the molten steel segregates in a region where the solidification is delayed, and inconveniences such as deterioration of central segregation occur, so that a difference in the solidification position in the width direction becomes an important quality index. Therefore, when a solidification position shape suggesting that a part of solidification is delayed is obtained, it is determined as a fail as quality nonconformance.

Note that as a result of continuous measurement, feedback to an operating process is possible. The feedback in real time is also possible, such as changing a cooling condition such as changing a secondary cooling pattern, adjusting the casting speed, the opening degree pattern of the casting segment, and a soft reduction condition. As so-called dynamic control of casting, it is possible to perform high-quality casting by feedback-controlling the solidification position to the operating conditions. When there is a problem in quality due to, for example, the measured solidification position shape exceeding the prescribed range, it is possible to immediately control the casting and operating conditions to proper casting and operating conditions, and there is an expectation for improvement in quality and improvement in yield.

In addition, slabs having quality problems have been subjected to destination change or scrapping in the past. However, these measures lower the yield and have a great impact on cost. Then, in the conventional measurement and slab quality assurance, since manual measurement or spot measurement has been performed, the assurance in units of slabs has been difficult, and a large amount of nonconformance has occurred such that all slabs are nonconforming in each charge and in each continuous-continuous casting. On the other hand, by performing the determination in units of slabs, the casting conditions can be immediately changed and corrected.

Further, even for slabs having quality problems, the influence can be minimized by changing post-process processing. As an example, when the solidification position is inappropriate, central segregation or porosity is suspected. When a slab having a solidification position deviating from a prescribed position is generated, quality improvement may be achieved by adding a heat treatment process or a forging process. In particular, the forging process is a process having an effect of rendering a void (porosity) in the central area harmless by strong reduction, but is costly because it is an additional process. On the other hand, by using the result of the measurement of the solidification position and applying it only to a slab having a problem, it is possible to achieve both cost and yield. In addition, it is possible to take measures such as performing ultrasonic flaw detection of the slab and more precisely performing quality evaluation and determination.

Conventionally, it has not been possible to automatically measure the solidification position due to factors such as a fluctuation in the thickness of the slab, measurement frequency has been low due to manual traveling measurement, it has not been possible to associate shapes with all slabs, and there has been a restriction that a measurement example in a steady state of casting is used as a representative of the casting. Therefore, it has not been possible to cope with unexpected acceleration/deceleration, a change in operating conditions, and the like. As a result, quality nonconforming slabs have been sent to the next process, or they have been excluded (or appropriated to the lower grade) more than necessary for safety.

On the other hand, by automating the measurement according to the embodiment, it is possible to reliably grasp the solidification position and solidification position shape with respect to the every slab and to perform the pass/fail determination. As a result, it is possible to improve slab internal quality, improve the yield, and suppress nonconformance.

Note that although the solidification position shape obtained in the embodiment has some irregularities, it has been confirmed that the solidification position is present within a management range. A result consistent with other quality indexes such as actual slab macro has been obtained, and it has been confirmed that there is no problem in quality. Note that the time required for the measurement has been about four and a half minutes for one set. When 17-point measurement one measurement 6 seconds = 102 seconds, movement 10 seconds $\times$ 16 travels = 160 seconds, total 262 seconds (4 minutes 22 seconds), and a casting speed of 1.1 m/min, one set of measurement is 4.8 m, which is within a range of the length of the slab, and can be associated with one slab. In a conventional solidification position measuring apparatus of a manual type, it has taken about 20 minutes to measure the solidification position in the width direction, and it has been impossible to determine the solidification position for each slab.

<Concept of Solidification position measuring apparatus>

Next, a concept of the solidification position measuring apparatus will be described. Conventionally, the reason why it has been difficult to continuously and automatically measure the solidification position and the solidification state of the cast-piece has been that there is a high possibility of causing serious troubles such that the electromagnetic ultrasonic sensor and the cast-piece come into contact with each other due to a fluctuation in the thickness of the cast-piece or the like, resulting in burning and damage, or the electromagnetic ultrasonic sensor is broken and caught in a roll. Lift-off, which is a distance between the cast-piece and the electromagnetic ultrasonic sensor, is usually about 4 mm to 6 mm, and contact easily occurs due to changes in the thickness of the cast-piece and the degree of roll opening. Conversely, if the lift-off increases measurement becomes impossible in some cases, for example, when the thickness of the cast-piece becomes thin or the boom supporting the electromagnetic ultrasonic sensor is thermally deformed.

Then, the thickness of the cast-piece is different for each operation. In general, casting and operation are performed according to nominal cast-piece thicknesses of 220 mm, 250 mm, and 300 mm, but the opening degree pattern of the cast segment, the soft reduction condition, the cooling condition, and the casting speed are different for each steel type or manufacturing condition, and the degree of solidification shrinkage is also different for each steel type. As a result, even with the same nominal cast-piece thickness, there is a thickness difference of about several millimeters in the actual thickness.

In addition, operation is generally performed in which the thickness is increased by several millimeters with respect to the final slab thickness at the middle stage of casting, then the opening degree is gradually reduced by segment opening degree control or the like in consideration of soft reduction or solidification shrinkage, and the thickness is controlled to the final slab thickness. However, during the operation of continuous casting, rapid deceleration or a temporary stop of casting may occur due to various factors and operating troubles, such as ladle change, deceleration for inserting a partition metal for different steel-type continuous-continuous casting in which different steel types are continuously cast, and deceleration due to a sign of a breakout. At the time of such rapid deceleration or stopping of casting, solidification may occur on the upstream side of the continuous casting facility. As a result, the cast-piece may be several millimeters thicker than that expected. This is because, since solidification has already been completed to the inside, the thickness cannot be reduced to a prescribed thickness by the reduction by the casting segment, and when the casting is resumed or the casting speed returns to a normal casting speed, this thickened portion is pulled out as it is. Then, there is a possibility that the thickened portion reaches the sensor position and comes into contact with the cast-piece.

Thus, the lift-off of the electromagnetic ultrasonic sensor is typically 4 to 6 mm. Due to the fluctuation in the cast-piece thickness or the like, there are risks of contact with the cast-piece, sensor damage, or being caught.

Therefore, for the fluctuation in the cast-piece thickness, constant monitoring or visually monitoring the roll opening degree of the casting segment or the set cast-piece thickness has been performed, to perform electromagnetic measurement. Since the measurement includes manual operation, it has not been possible to continuously perform the measurement, and it has not been realistic to perform the measurement for the entire operation and the entire slab.

Although it is conceivable to provide the gap measuring unit for measuring the distance between the cast-piece and the electromagnetic ultrasonic sensor, there has been no distance sensor that can be installed between casting rolls, is resistant to heat, can be used for a long period of time in a bad environment such as steam, oil mist, grease, scale, dust, and the like, and has an appropriate measurement range and accuracy.

In addition, continuous casting is a facility for producing a cast-piece by continuously injecting molten steel into a mold and pulling out the molten steel, but in the final stage of continuous casting, the injection of the molten steel is stopped and the molten steel is pulled out. At this time, since the static iron pressure is eliminated, the cast-piece becomes thin. When the cast-piece becomes thin, the lift-off becomes large, the efficiency of transmission/reception decreases, and the sensitivity of transmission/reception of electromagnetic ultrasonic waves decreases.

Further, the surface of the cast-piece has a very high temperature of around 1000°C, and the boom receives strong radiant heat. In addition, since the boom needs to travel in a narrow gap such as a gap of the cast segment, it is necessary to make the boom thin, and it is necessary to keep a sufficient size in the height direction in order to maintain rigidity. Therefore, a temperature difference occurs between the upper and lower sides of the boom, and a difference in thermal expansion occurs. In general, since the cast-piece side is heated to a high temperature, the cast-piece side is elongated, and thermal expansion occurs in a direction in which the lift-off of the electromagnetic ultrasonic sensor becomes large. If the height is set based on the cast-piece thickness or the like, due to this boom warpage the lift-off increases more than a reference or an assumed one, the measurement sensitivity decreases, and the accuracy decreases due to deterioration of the SN, or the measurement cannot be performed at all.

In the embodiment, for the cast-piece, the electromagnetic ultrasonic sensor is provided on the boom capable of lifting and lowering and moving transversely by the sensor lifting and lowering unit and the sensor traveling unit, the gap measuring unit for measuring the gap between the cast-piece and the electromagnetic ultrasonic sensor is provided, the gap between the cast-piece and the electromagnetic ultrasonic sensor is constantly measured by the gap measuring unit, the position of the electromagnetic ultrasonic sensor is controlled, a state in which measurement is impossible due to contact between the electromagnetic ultrasonic sensor and the cast-piece, damage, or a decrease in sensitivity due to a lift-off fluctuation is prevented, and it is possible to perform continuous measurement and continuous evaluation of the solidification position of the cast-piece.

<Metal Material Quality Management Method>

The solidification position measuring method according to the above embodiment can also be applied to, for example, a metal material quality management method. Then, in the metal material quality management method, the quality of a metal material is managed based on the solidification position measurement result, such as controlling manufacturing conditions on the next process from the solidification position of the cast-piece measured using the solidification position measuring method.

<Casting Facility and Metal Material Manufacturing Facility>

The solidification position measuring apparatus according to the above embodiment can also be applied to, for example, a casting facility such as a continuous casting facility. Then, in a metal material manufacturing facility including the casting facility, a metal material can be manufactured from a cast-piece manufactured by the casting facility. Here, the casting facility refers to both a case where there is only one device for casting a metal material and a case where a plurality of the devices are combined.

In addition, the solidification position measuring apparatus according to the above embodiment can also be applied to a metal material manufacturing facility. Then, in the metal material manufacturing facility, a metal material can be manufactured while the solidification position of the cast-piece is measured by the solidification position measuring apparatus.

<Metal Material Manufacturing Method>

The solidification position measuring method according to the above embodiment can also be applied to, for example, a metal material manufacturing method. Then, in the metal material manufacturing method, a metal material is manufactured while the solidification position of the cast-piece is measured using the solidification position measuring method. As a result, the metal material having a preset quality can be manufactured.

According to the solidification position measuring apparatus, the solidification position measuring method, the metal material quality management method, the casting facility, the metal material manufacturing facility, and the metal material manufacturing method according to the embodiment described above, it is possible to continuously and automatically measure the solidification position of the cast-piece by preventing damage and burning of the electromagnetic ultrasonic sensor due to a decrease in lift-off caused by a fluctuation in the thickness of the cast-piece and preventing a decrease in sensitivity of the electromagnetic ultrasonic sensor due to an increase in lift-off.

Note that in the present invention, the above effects can be reliably obtained in a case where the "metal material" is a "steel material", which is most preferable. In addition, the above effects can be maximized in a case where the "casting facility" is the "continuous casting facility", which is most preferable.

Hereinabove, the solidification position measuring apparatus, the solidification position measuring method, the metal material quality management method, the metal material manufacturing facility, and the metal material manufacturing method according to the present invention have been specifically described by the embodiment and examples for carrying out the invention, but the gist of the present invention is not limited to these descriptions, and should be widely interpreted based on the description of the claims. In addition, it goes without saying that various changes, modifications, and the like based on these descriptions are also included in the gist of the present invention.

Reference Signs List

> 1, 1A CAST-PIECE
> 2 SOLIDIFIED PORTION
> 3 LIQUID PHASE PORTION
> 4 SOLIDIFICATION POSITION (CRATER END POSITION)
> 6, 6a, 6b WATER COLUMN DISTANCE METER
> 7, 7a, 8 ELECTROMAGNETIC ACOUSTIC TRANSDUCER
> 10 SOLIDIFICATION POSITION MEASURING APPARATUS
> 11, 12 BOOM
> 13 DRIVE CONTROL UNIT
> 14 MEASUREMENT COMPUTING UNIT
> 15 TRANSMISSION UNIT
> 21 to 24 MOTOR
> 30 MAGNET
> 31 AUXILIARY MAGNET
> 41 LONGITUDINAL WAVE COIL
> 42 TRANSVERSE WAVE COIL
> 61 ULTRASONIC PROBE
> 62 WATER SUPPLY PORT
> 63 WATER TANK
> 64 NOZZLE
> 65 STEP
> 66 WATER COLUMN
> 101 MOLD
> 102 CAST-PIECE SUPPORT ROLL
> 103 CONVEYING ROLL
> 104 CAST-PIECE CUTTING MACHINE
> 110 HOST DEVICE
> L1 to L3 WAVEFORM
> T1, T2 TIME
> $\Delta t$ PROPAGATION TIME

**Claims**

1. A solidification position measuring apparatus for measuring a solidification position of a cast-piece, the solidification position measuring apparatus comprising:

> an electromagnetic acoustic transducer installed outside the cast-piece;
> a sensor lifting and lowering unit configured to adjust a gap between the cast-piece and the electromagnetic acoustic transducer;
> a gap measuring unit configured to measure the gap between the cast-piece and the electromagnetic acoustic transducer;
> a computing unit configured to estimate the solidification position in the cast-piece from an output of the electromagnetic acoustic transducer; and
> a drive control unit configured to drive and control the sensor lifting and lowering unit and the gap measuring unit, wherein
> the drive control unit is configured to control a position of the electromagnetic ultrasonic sensor by the sensor lifting and lowering unit based on the gap measured by the gap measuring unit.

2. The solidification position measuring apparatus according to claim 1, further comprising
a sensor traveling unit configured to move, outside the cast-piece, the electromagnetic ultrasonic sensor in a plane parallel to a horizontal plane of the cast-piece.

3. The solidification position measuring apparatus according to claim 1 or 2, wherein
the gap measuring unit is configured to shoot water to a measurement target, propagate an ultrasonic wave in the shot water, and measure a distance from ultrasonic wave generating unit to the measurement target based on a propagation time of a reflection signal of the ultrasonic wave.

4. The solidification position measuring apparatus according to any one of claims 1 to 3, wherein

a transmission/reception signal of the electromagnetic ultrasonic sensor is a chirp signal whose frequency changes with time, and
the computing unit is configured to perform computing processing including a phase operation on the chirp signal.

5. The solidification position measuring apparatus according to claim 4, wherein
the computing unit is configured to detect, after the computing processing, a waveform of a transmitted wave and/or a reflected wave at a time gate including the transmitted wave and/or the reflected wave, and compute a propagation time using a time difference of the transmitted wave and/or the reflected wave whose waveform is detected.

6. The solidification position measuring apparatus according to any one of claims 1 to 5, wherein
the electromagnetic ultrasonic sensor is configured to use a Halbach array magnet.

7. A solidification position measuring method for measuring a solidification position of a cast-piece, the solidification position measuring method comprising:

a gap measuring step of measuring a gap between the cast-piece and an electromagnetic ultrasonic sensor;
a gap determining step of determining whether or not the gap measured is within a prescribed range;
a gap adjusting step of adjusting the gap based on the gap measured when the gap is not within the prescribed range; and
a computing step of estimating the solidification position in the cast-piece using the electromagnetic ultrasonic sensor when the gap is within the prescribed range.

8. The solidification position measuring method according to claim 7, further comprising
a moving step of moving the electromagnetic ultrasonic sensor in a plane parallel to a horizontal plane of the cast-piece.

9. The solidification position measuring method according to claim 7 or 8, wherein

the electromagnetic ultrasonic sensor is configured to use a Halbach array magnet,
a transmitting /receiving signal of the electromagnetic ultrasonic sensor is a chirp signal whose frequency changes with time, and
the computing step performs computing processing including a phase operation on the chirp signal.

10. A metal material quality management method comprising
controlling a manufacturing condition of a next process from a solidification position of a cast-piece measured using the solidification position measuring method according to any one of claims 7 to 9.

11. A metal material manufacturing facility comprising

the solidification position measuring apparatus according to any one of claims 1 to 6, wherein
a metal material is manufactured while the solidification position of the cast-piece is measured by the solidification position measuring apparatus.

12. A casting facility comprising

the solidification position measuring apparatus according to any one of claims 1 to 6, wherein
a cast-piece is manufactured while the solidification position of the cast-piece is measured by the solidification position measuring apparatus.

**13.** A metal material manufacturing facility comprising

the casting facility according to claim 12, wherein
a metal material is manufactured from a cast-piece manufactured by the casting facility.

**14.** A metal material manufacturing method comprising
manufacturing a metal material while measuring a solidification position of a cast-piece using the solidification position measuring method according to any one of claims 7 to 9.

# FIG.1

FIG.2

10

110 HOST DEVICE

13 DRIVE CONTROL UNIT

21
M

11 BOOM

23 M

22 M

12 BOOM

7

6

8

1

14 CALCULATION COMPUTING UNIT

15 TRANSMISSION UNIT

Z
Y
X

# FIG.3

```
        ( START )
            │
            ▼                    ⌐S11
  ┌───────────────────────┐
  │  PREPARE MEASUREMENT   │
  └───────────────────────┘
            │
            ▼                    ⌐S12
  ┌───────────────────────┐
  │  CAUSE BOOM TO TRAVEL  │
  └───────────────────────┘
            │
            ▼                    ⌐S13
      NO  ◇ IS MOVEMENT ◇
  ◄───────   COMPLETED?
            │
           YES
            │
            ▼                    ⌐S14
  ┌───────────────────────┐
  │ ACQUIRE INDICATION     │
  │ VALUE OF WATER COLUMN  │
  │ DISTANCE METER         │
  └───────────────────────┘
            │
            ▼                    ⌐S15
      ◇ IS LIST-OFF VALUE ◇   NO      ⌐S16
        WITHIN PRESCRIBED ──────►┌──────────────────┐
           RANGE?                │ ADJUST LIST-OFF  │
            │                    └──────────────────┘
           YES    ⌐S17
            │
            ▼
  ┌───────────────────────┐
  │ MEASURE LONGITUDINAL   │
  │ WAVE AND MEASURE       │
  │ TRANSVERSE WAVE BY     │
  │ ELECTROMAGNETIC        │
  │ ACOUSTIC TRANSDUCER    │
  └───────────────────────┘
            │
            ▼                    ⌐S18
  ┌───────────────────────┐
  │ ESTIMATE AND COMPUTE   │
  │ SOLIDIFICATION POSITION│
  └───────────────────────┘
            │
            ▼                    ⌐S19
    YES   ◇ IS THERE ◇
  ◄─────── MOVEMENT
            INSTRUCTION?
            │
            NO
            │
            ▼
        ( END )
```

23

# FIG.4

START

↓ S21

PREPARE MEASUREMENT

↓ S22

CAUSE BOOM TO TRAVEL

↓ S23

NO ← IS MOVEMENT COMPLETED?

↓ YES

↓ S24

ACQUIRE INDICATION VALUE OF WATER COLUMN DISTANCE METER

↓ S25

IS LIST-OFF VALUE WITHIN PRESCRIBED RANGE? → NO

↓ YES ↓ S26

ADJUST LIST-OFF

↓ S27

MEASURE LONGITUDINAL WAVE AND MEASURE TRANSVERSE WAVE BY ELECTROMAGNETIC ACOUSTIC TRANSDUCER

↓ S28

ESTIMATE AND COMPUTE SOLIDIFICATION POSITION

↓ S29

YES ← IS THERE NEXT MEASUREMENT INSTRUCTION?

↓ NO

END

# FIG.5

# FIG.6

# FIG.7

COOLING WATER

SIGNAL CABLE

ELECTROMAGNETIC ACOUSTIC TRANSDUCER

# FIG.8

7

30  30  30

MAGNET

LONGI-
TUDINAL
WAVE COIL

41

LORENTZ FORCE

MAGNETIC FLUX

EDDY
CURRENT

1

COIL:
PERPENDICULAR
TO PAPER SURFACE
INDUCED CURRENT:
PERPENDICULAR
TO PAPER SURFACE

ULTRASONIC WAVE
(LONGITUDINAL WAVE)

LONGITUDINAL WAVE:
USING HORIZONTAL COMPONENT OF
MAGNETIC FIELD

# FIG.9

7

30  30  30

MAGNET

TRANS-
VERSE
WAVE COIL

42

LORENTZ FORCE

MAGNETIC FLUX

EDDY
CURRENT

1

COIL:
PERPENDICULAR
TO PAPER SURFACE
INDUCED CURRENT:
PERPENDICULAR
TO PAPER SURFACE

ULTRASONIC WAVE
(TRANSVERSE WAVE)

TRANSVERSE WAVE:
USING VERTICAL COMPONENT OF
MAGNETIC FIELD

## FIG.10

30            30            30

| S | | N | | S |
| | | | | |
| N | | S | | N |

LONGITUDINAL WAVE COIL
TRANSVERSE WAVE COIL

41

42

## FIG.11

30      31      30      31      30

| S | S   N | N | N   S | S |
| | | | | |
| N | | S | | N |

LONGITUDINAL WAVE COIL
TRANSVERSE WAVE COIL

41

42

# FIG.12

L1: —————ONLY HALBACH

L2: ━━━━━ HALBACH (CHIRP PULSE COMPRESSION IS PRESENT)

# FIG.13

L2: ————— HALBACH (CHIRP PULSE COMPRESSION IS PRESENT)

L3: ━━━━━ CONVENTIONAL HEAD (CHIRP PULSE COMPRESSION IS PRESENT)

# FIG.14

(a)

TRANSVERSE WAVE
TRANSMISSION

TRANSVERSE WAVE IS PRESENT=COMPLETE SOLIDIFICATION

(b)

TRANSVERSE
WAVE IS
ABSENT

TRANSVERSE WAVE IS ABSENT=NON-SOLIDIFICATION

# FIG.15

# FIG.16

(a)

FLUCTUATE DEPENDING
ON CASTING SPEED

ONE SET

WIDTH MEASUREMENT PITCH

Z ⊙ → Y

X

○ MEASUREMENT
POINT

CASTING
DIRECTION ⇒

ONE SET

(b)

SOLIDIFICATION POSITION

# FIG.17

(a)

(b)

## INTERNATIONAL SEARCH REPORT

| International application No. |
| --- |
| **PCT/JP2023/005639** |

**A.    CLASSIFICATION OF SUBJECT MATTER**

***B22D 11/16*** (2006.01)i; ***G01B 17/00*** (2006.01)i
FI:    B22D11/16 104S; G01B17/00 B

According to International Patent Classification (IPC) or to both national classification and IPC

**B.    FIELDS SEARCHED**

Minimum documentation searched (classification system followed by classification symbols)

B22D11/16; G01B17/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2019-162664 A (KOBE STEEL, LTD.) 26 September 2019 (2019-09-26) claims, paragraph [0038] | 1-14 |
| A | JP 2002-014083 A (NKK CORP.) 18 January 2002 (2002-01-18) entire text, all drawings | 1-14 |
| A | JP 2015-62918 A (JFE STEEL CORP.) 09 April 2015 (2015-04-09) entire text, all drawings | 1-14 |

☐ Further documents are listed in the continuation of Box C.          ☑ See patent family annex.

| | |
| --- | --- |
| *    Special categories of cited documents: <br> "A"   document defining the general state of the art which is not considered to be of particular relevance <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
| --- | --- |
| **11 April 2023** | **25 April 2023** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT
### Information on patent family members

International application No.

**PCT/JP2023/005639**

| Patent document cited in search report | | | Publication date (day/month/year) | Patent family member(s) | | | Publication date (day/month/year) |
|---|---|---|---|---|---|---|---|
| JP | 2019-162664 | A | 26 September 2019 | (Family: none) | | | |
| JP | 2002-014083 | A | 18 January 2002 | US | 2003/0141036 | A1 | |
| | | | | entire text, all drawings | | | |
| | | | | US | 2005/0161190 | A1 | |
| | | | | US | 2007/0051486 | A1 | |
| | | | | WO | 2002/090971 | A1 | |
| | | | | EP | 1298429 | A1 | |
| | | | | EP | 1666173 | A1 | |
| | | | | EP | 1900454 | A2 | |
| | | | | BR | 111910 | A | |
| | | | | TW | 506866 | B | |
| | | | | KR | 10-2005-0085988 | A | |
| | | | | KR | 10-2005-0085989 | A | |
| | | | | CN | 1437704 | A | |
| | | | | KR | 10-0594858 | B1 | |
| JP | 2015-62918 | A | 09 April 2015 | (Family: none) | | | |

Form PCT/ISA/210 (patent family annex) (January 2015)

**EP 4 470 693 A1**

**Patent documents cited in the description**

- JP 2010005700 A

- JP H10122844 A